# EUROPEAN PATENT SPECIFICATION

(11) **EP 2 516 677 B1**
(45) Date of publication and mention of the grant of the patent: **26.11.2014**
(21) Application number: 10838414.0
(22) Date of filing: 22.12.2010
(51) Int. Cl.: C12Q 1/68

(54) **METHODS OF ENRICHING AND DETECTING FETAL NUCLEIC ACIDS**
VERFAHREN ZUR ANREICHERUNG UND DETEKTION FETALER NUCLEINSÄUREN
PROCÉDÉS D'ENRICHISSEMENT ET DE DÉTECTION D'ACIDES NUCLÉIQUES FOETAUX

(30) Priority: 23.12.2009 US 289710 P
(43) Date of publication of application: 31.10.2012
(73) Proprietor: Genetic Technologies Limited, Fitzroy, VIC 3065 (AU)
(72) Inventor: MANTZARIS, Debbie, Avondale Heights Victoria 3034 (AU); ALLMAN, Richard, Wyndham Vale Victoria 3024 (AU); CANTSILIERIS, Stuart, Melbourne Victoria 3004 (AU); VOM, Eduardo, Richmond Victoria 3121 (AU); ASHDOWN, Maria Luisa, Thornbury Victoria 3071 (AU)
(74) Representative: Docherty, Robert Charles
(86) International application number: PCT/AU2010/001718
(87) International publication number: WO 2011/075774

(56) References cited:
- EP-A1- 0 363 196
- WO-A1-2010/085841
- WO-A2-2008/070862
- WO-A2-2008/081451
- WO-A2-2009/102632
- US-A- 6 059 735
- HOLZGREVE W ET AL: "Fetal cells in cervical mucus and maternal blood", BAILLIERE'S BEST PRACTICE AND RESEARCH. CLINICAL OBSTETRICS ANDGYNAECOLOGY, BAILLIERE TINDALL, LONDON, GB, vol. 14, no. 4, 1 January 2000 (2000-01-01), pages 709-722, XP008081646, ISSN: 1521-6934, DOI: 10.1053/BEOG.1999.0106

## Description

### FIELD OF THE INVENTION

The present invention relates to a method of enriching free fetal nucleic acids from a cervical sample. The enriched fetal nucleic acids can be used in a variety of procedures including, detection of a trait of interest such as a disease trait, or a genetic predisposition thereto, gender typing and parentage testing.

### BACKGROUND OF THE INVENTION

Currently, fetal diagnosis is generally carried out by a procedure known as amniocentesis which involves the aspiration of a small sample of amniotic fluid from the pregnant mother, culturing the fetal cells in the fluid, and determining the karyotype of the fetal cells. Recently, chorionic villus sampling has also been used, which involves the direct transcervical and transabdominal aspiration of the chorionic villus. However, as both amniocentesis and chorionic villus sampling require invasive procedures for obtaining fetal cells, they inevitably expose both the mother and the fetus to a certain amount of risk. Accordingly non-invasive approaches to prenatal diagnosis are preferred.

Cell-free fetal DNA circulates in the plasma of pregnant women (Lo *et al*., 1997). Up to at least 7% of all cell-free DNA in maternal plasma/serum has been found to be of fetal origin when using RT-PCR analysis. It has been demonstrated that this fetal DNA can be used to predict the gender of the fetus (Lo *et al*., 1997), as well as to determine the fetal Rhesus D status (Lo *et al*., 1998a). Studies have also indicated that the amount of fetal DNA in maternal plasma/serum can be correlated with certain fetal abnormalities such as trisomy 21 (Down syndrome) (Lee *et al*., 2002) and trisomy 13 (Wataganara *et al.,* 2003).

Holzgreve and Hahn (2000) disclose alternative methods to amniocentesis or chorionic villus sampling by isolating fetal cells from the blood of pregnant women or trophoblast cells from the cervix. The authors describe moreover that free extracellular fetal DNA can be detected in the plasma and serum of pregnant women which is suitable for the accurate detection of fetal loci such as the fetal rhesus D gene. However, the broad application of both methods for the detection of various abnormalities is limited by the low abundance of trophoblast or fetal DNA in the sample and therefore effective enrichment methods are necessary.

It has also been demonstrated that cell-free fetal DNA may be found in the amniotic fluid of pregnant women (Bianchi *et al*., 2001). The size range of this DNA appears to be similar to that found circulating in the maternal circulation, although the size "distribution" has been reported to vary in the case of aneuploid pregnancies (Peter, *et al*., 2008; WO 05/044086; and WO 07/027970).

There is a need for alternate methods of obtaining enriched fetal nucleic acids from a pregnant female.

### SUMMARY OF THE INVENTION

The present inventors have surprisingly found free fetal nucleic acids in samples obtained from the cervix. The inventors were particularly surprised to find that the average concentration of fetal nucleic acids in cervical samples is considerably higher than for plasma or serum samples.

In one aspect, the present invention provides a method of enriching fetal nucleic acids from a pregnant female, the method comprising
i) obtaining a cervical sample, and
ii) enriching extracellular nucleic acids comprising fetal nucleic acids by separating cells and/or cell nuclei from the extracellular nucleic acids in the sample.

Fetal nucleic acids enriched using a method of the invention can be used to analyse the genotype of the fetus. Thus, in another aspect the present invention provides a method for analysing the genotype of a fetus at a locus of interest, the method comprising
i) obtaining a cervical sample,
ii) separating cells and/or cell nuclei from the extracellular nucleic acids comprising fetal nucleic acids in the sample, and
iii) analysing the fetal nucleic acids for the genotype of a fetus at a locus of interest.

The genotype of the fetus can be determined using any technique known in the art. Examples include, but are not limited to, hybridization based procedures and/or amplification based procedures.

The genotype of fetal nucleic acids can be analysed for any purpose. Typically, the genotype will be analysed to detect the likelihood that the offspring will possess a trait of interest. Preferably, the fetal nucleic acids are analysed for a genetic abnormality linked to a disease state, or predisposition thereto. In one embodiment, the genetic abnormality is in the structure and/or number or chromosomes. In another embodiment, the genetic abnormality encodes an abnormal protein. In another embodiment, the genetic abnormality results in decreased or increased expression levels of a gene.

The enriched fetal nucleic acids can be used to determine the sex of the fetus. As a result, in a further aspect, the present invention provides a method of determining the sex of a fetus, the method comprising
i) obtaining a cervical sample,
ii) separating cells and/or cell nuclei from the extracellular nucleic acids comprising fetal nucleic acids in the sample, and
iii) analysing the fetal nucleic acids to determine the sex of the fetus.

The analysis of the enriched fetal nucleic acids to determine the sex of the fetus can be performed using any technique known in the art. For example, Y-chromosome specific probes can be used.

The enriched fetal nucleic acids can also be used to identify the father of the fetus. Accordingly, in a further aspect, the present invention provides a method of determining the father of a fetus, the method comprising
i) obtaining a cervical sample,
ii) separating cells and/or cell nuclei from the extracellular nucleic acids comprising fetal nucleic acids in the sample,
iii) determining the genotype of the candidate father at one or more loci,
iv) analysing the fetal nucleic acids to determine the genotype of the fetus at one or more of said loci, and
v) comparing the genotypes of iii) and iv) to determine the probability that the candidate father is the biological father of the fetus.

The present inventors have found that fetal nucleic acids obtained using the methods of the invention are larger than the free fetal nucleic acids obtained by other means such as from blood or plasma. This means that nucleic acids obtained using the methods of the invention are more suited to a variety of techniques including whole genome amplification. Thus, in a preferred embodiment, at least some of the fetal nucleic acids are greater than about 500 nucleotides in length. More preferably, at least 50% of the fetal nucleic acids are greater than about 500 nucleotides in length.

In a preferred embodiment, the cervical sample is a transcervical sample. In a preferred embodiment, the sample is obtained from between the internal os and the external os of the cervix. More preferably, the sample is obtained closer to the internal os of the cervix.

Whilst the present inventors have shown that a number of devices can be used in the methods of the invention, the RareCellect™ device was found to have the optimal level of safety and efficiency. Accordingly, in a particularly preferred embodiment, the sample is obtained using a device adapted for transcervical sampling of biological materials from a pregnant female, the device comprising:
- an elongate insertion tube having a first end adapted for insertion through the external orifice (external os) of the cervix and a second end including a handle means for manipulating said tube;
- a measuring means for determining the position of said first end of said tube within the cervix,
wherein said first end includes a sampling head adapted to collect biological material including extracellular nucleic acids and mucus, and wherein said measuring means is adapted to determine the extent of insertion and transcervical position of said first end to optimise the transcervical sampling site.

The cells and/or cell nuclei are separated from the extracellular nucleic acids by any means known in the art. In a preferred embodiment, the cells and/or cell nuclei are separated from the extracellular nucleic acids by centrifugation.

Preferably, the sample is/was obtained within 5 to 18 weeks of pregnancy, more preferably within 5 to 15 weeks of pregnancy, and even more preferably within 5 to 12 weeks of pregnancy.

In an embodiment, the method further comprises obtaining the sample from the pregnant female.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention is hereinafter described by way of the following non-limiting Examples and with reference to the accompanying figures.

### BRIEF DESCRIPTION OF THE ACCOMPANYING DRAWINGS

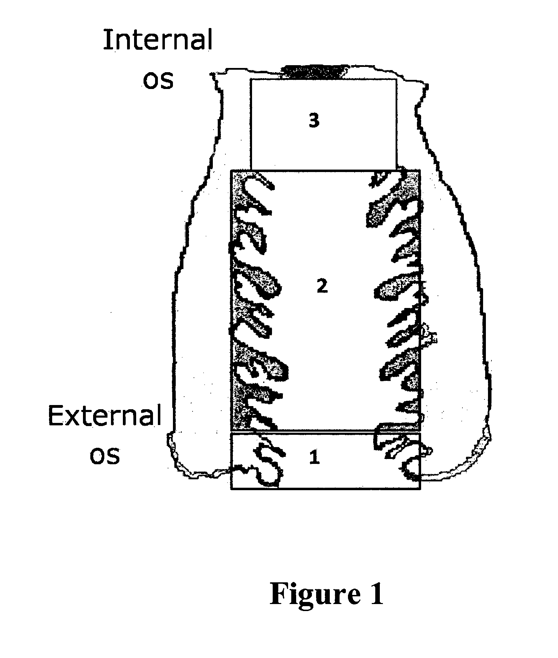

**Figure 1** **-** Schematic drawing of the cervix. The preferred site for obtaining a suitable transcervical sample is highlighted in region 3, closest to the internal os level. Region 1 represents the point of entry into the cervix at the external os level and region 2 is the body of the cervix (otherwise referred to as the endocervical canal).

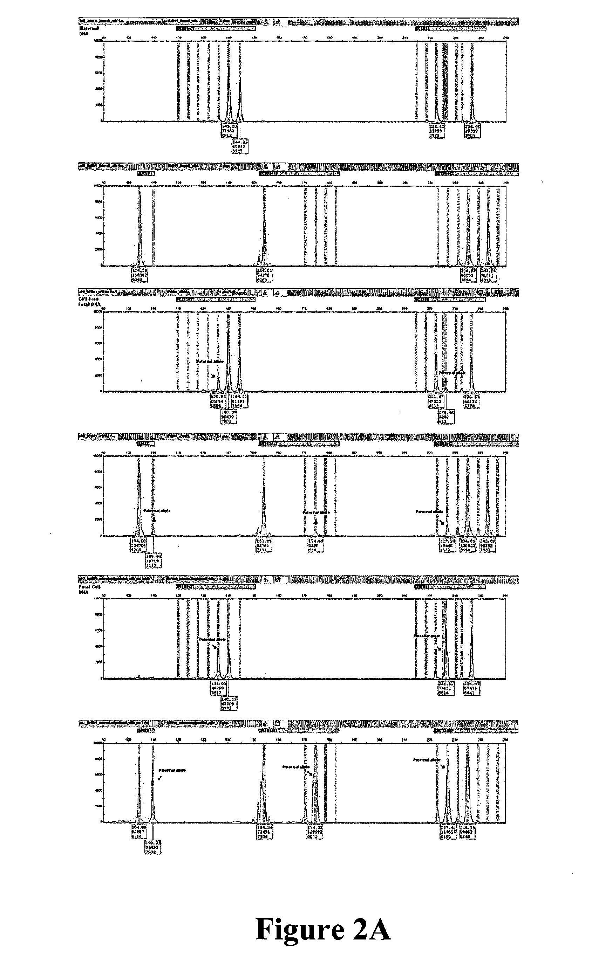

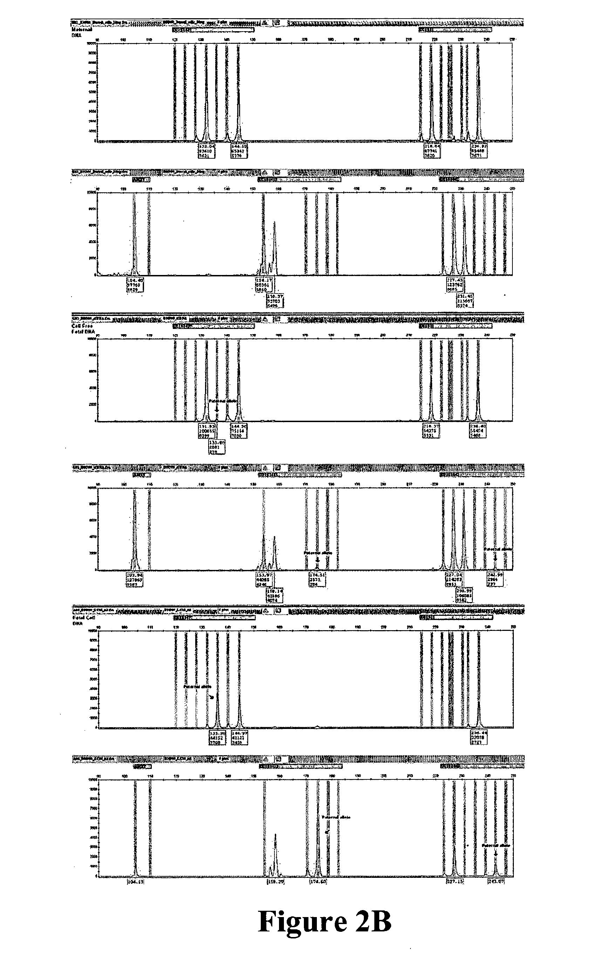

**Figure 2** **-** Electrophoretogram of amplified MF-PCR products from cell free DNA collected from cervical supernatant samples using four chromosome 21 STR markers and the sex marker, amelogenin. The x axis shows the calculated length of the amplified STR amplicons in base pairs and the y axis shows fluorescent intensities in arbitrary units. Comparative analysis of maternal DNA STR profiles and cell free DNA STR profiles shows the detection of paternally inherited alleles (indicated by arrows) can be achieved from both male (Figure 2A, sample number DH893) and female (Figure 2B, sample number DH908) derived fetuses. Analysis of matching fetal (syncytiotrophoblasts) DNA confirmed corresponding paternal alleles and the sex of the fetus in the cell free DNA sample.

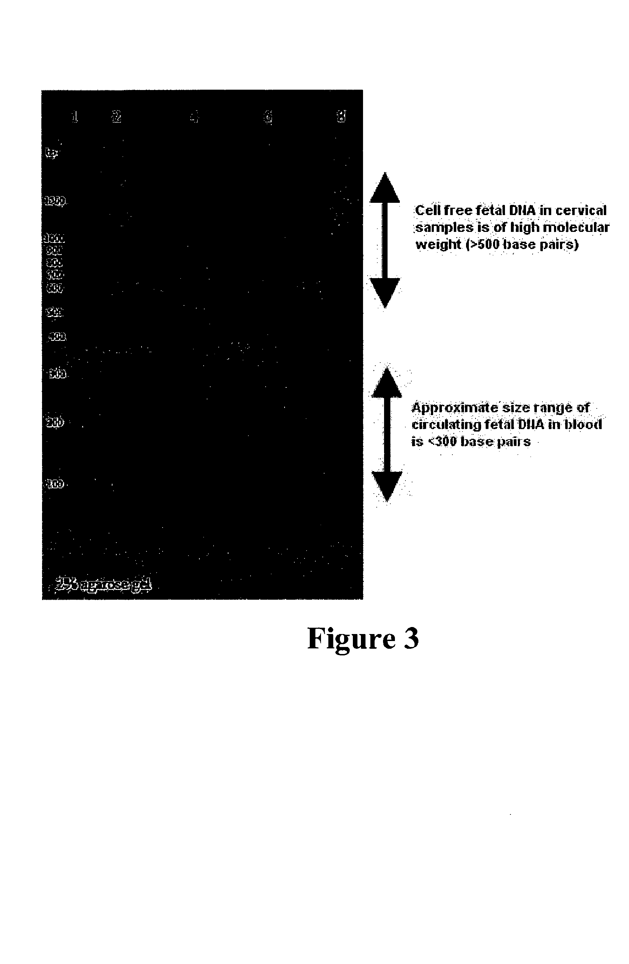

**Figure 3** **-** Agarose gel separation of cell free fetal DNA in cervical supernatant samples. Well 1=100-bp ladder; well 3= sample #DH979 - Cell free fetal DNA (110ng) -59.4% fetal; well 5= sample #DH945 - Cell free fetal DNA (202ng) - 20.9% fetal; and well 7= sample #DH984 Cell free DNA (94ng) - 9.6% fetal.

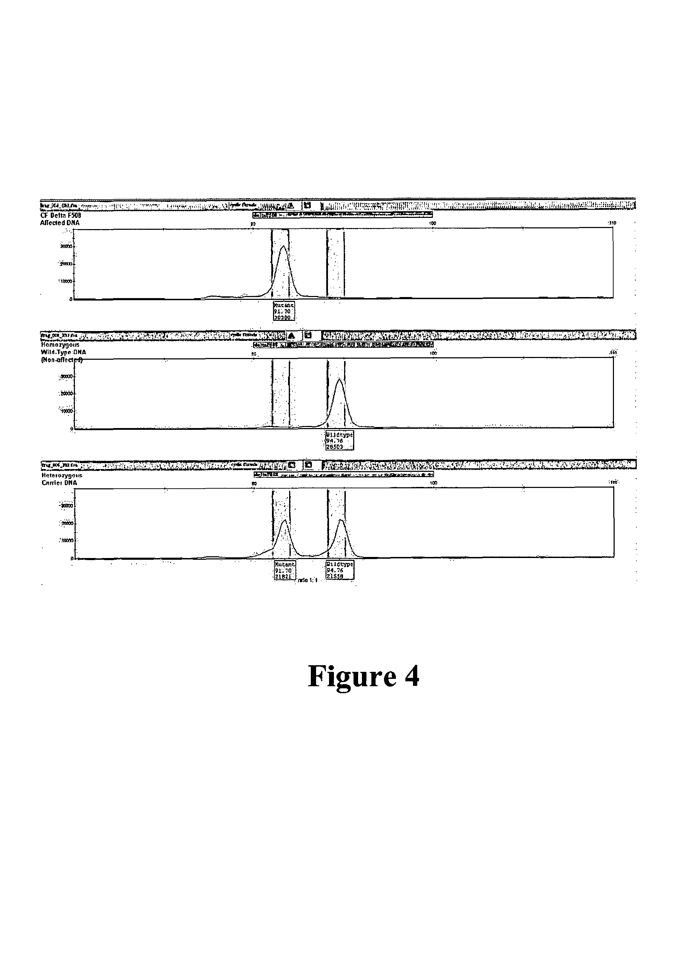

**Figure 4** **-** Fluorescent PCR based model system for testing of CF Delta F508 mutation.

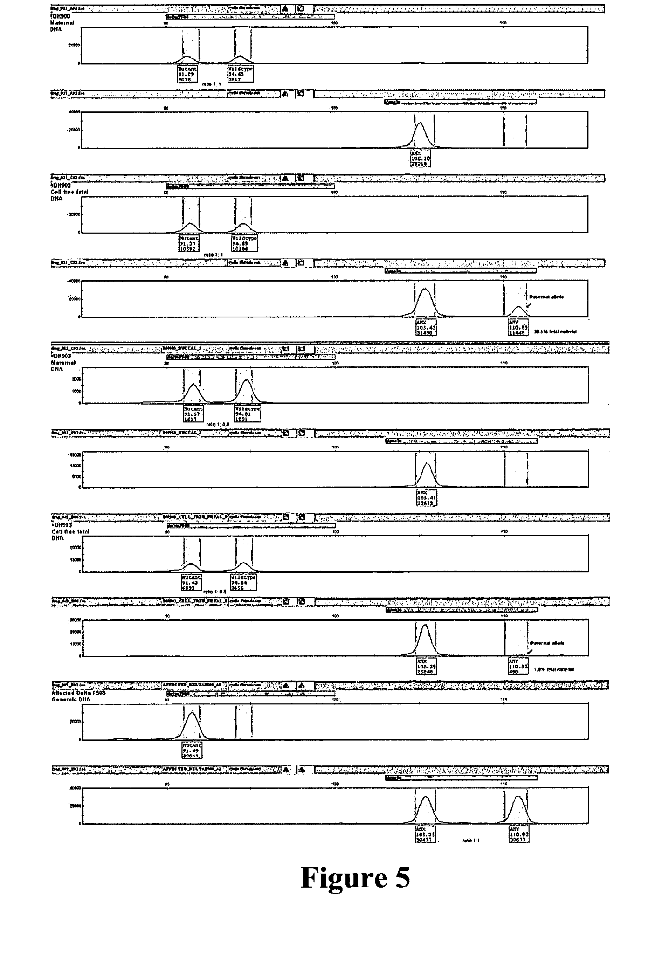

**Figure 5** **-** Electrophoretogram of amplified MF-PCR products fetal positive cell free DNA samples using the sex marker, amelogenin and the CF delta F508 marker. The x axis shows the calculated length of the amplified amplicons in base pairs and the y axis shows fluorescent intensities in arbitrary units. Comparative analysis of maternal DNA profiles and cell free DNA profiles showed that in both instances the fetal material in the cell free DNA samples #DH900 and #DH903 were derived from male fetuses. A carrier diagnosis for CF delta F508 mutation could only be reported for cell free DNA sample DH900 representing 37% fetal material of total sample.

### DETAILED DESCRIPTION OF THE INVENTION

### General Techniques and Definitions

Unless specifically defined otherwise, all technical and scientific terms used herein shall be taken to have the same meaning as commonly understood by one of ordinary skill in the art (e.g., fetal cell and DNA biology, prenatal diagnostics, molecular genetics, immunology, immunohistochemistry, protein chemistry, nucleic acid hybridization, flow cytometry, and biochemistry).

Unless otherwise indicated, the recombinant DNA and/or protein, cell culture, and immunological techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

As used herein, the terms "enriching", "enriched", "separating" and variations thereof are used in their broadest sense to encompass the isolation of the fetal nucleic acids such that the relative concentration of fetal nucleic acids to other biological material, particularly cells and nuclei, in the treated sample is greater than a comparable untreated sample. Preferably, the enriched fetal nucleic acids are separated from at least 10%, more preferably at least 20%, more preferably at least 30%, more preferably at least 40%, more preferably at least 50%, more preferably at least 60%, more preferably at least 70%, more preferably at least 75%, more preferably at least 80%, more preferably at least 90%, more preferably at least 95%, and even more preferably at least 99% of cells and nuclei in the sample obtained from the pregnant female. Most preferably, the enriched fetal nucleic acids contains no maternal nuclei or maternal cells. The terms "enrich" and variations thereof are used interchangeably herein with the term "isolate" and variations thereof.

As used herein, the term "extracellular nucleic acids" refers to nucleic acids in the sample, particularly as obtained from the female, which is not present within a cell and/or nuclei. Such nucleic acids are usually the result of cell death/lysis *in vivo.* The fetal nucleic acids can be DNA or RNA or generally a combination thereof. Preferably, the fetal nucleic acids at least comprise DNA. The free nucleic acids may be derived from, for example, the nucleous or the mitochondria.

### Sample

The term "sample" as used herein includes material taken directly from the female, as well as material that has already been partially purified. Examples of such partial purification include the removal of at least removal of maternal red blood cells, and/or removal of maternal lymphocytes. In a preferred embodiment, the sample is the material obtained from the female placed in a suitable storage and/or transport solution. The phrase "obtaining" the sample can include either physically collecting the sample from the female, or acquiring the sample, either directly or indirectly, from a source who has already collected the sample such as from a clinician.

As used herein, the term "cervical sample" refers to biological matter comprising both cellular and non-cellular taken from the exterior and/or interior of the cervix. For example, the cervical sample may be taken from the exterior surface of the cervix at the end of the vagina by scraping with a suitable device such as when used in when obtaining a pap smear. However, in a preferred embodiment the sample is a "transcervical sample". As used herein, the term "transcervical sample" refers to material taken from within the cervix of the pregnant female and comprises cervical mucous. In general, the cervix is 1 cm to 5 cm in length which can readily be determined by the skilled person using standard techniques such as ultrasound. In an embodiment, the sample is at least taken from the region of the cervix closest to the internal os (Figure 1).

The sample can be obtained using a variety of sampling methods including, but not limited to, aspiration, irrigation, lavage, adsorption and scraping. The sample may be obtained from sites including, but not limited to, the endocervical canal, external os, internal os, lower uterine pole and uterine cavity. A range of devices are available commercially which may be suitable for obtaining the sample, including but not limited to: "Aspiracath" aspiration catheter (Cook Medical, IN, USA), "Tao" brush endometrial sampler (Cook Medical, IN, USA), Goldstein Sonobiopsy catheter (Cook Medical, IN, USA), Aspiration kit (MedGyn, IL, USA), Endosampler (MedGyn, IL, USA), Endometrial sampler and cervical mucus sampling syringe (Rocket Medical, UK), "Sampling Probet" (Gynetics Products, Belgium), "Sampling in-out" - endometrial curette (Gynetics Products, Belgium), Endometrial cell sampler (Cheshire Medical Specialities Inc, CT, USA), Aspirette® Endocervical Aspirator and Embryo Transfer Catheter (Cooper Surgical, CT, USA), and Intrauterine Catheter (Cooper Surgical, CT, USA).

In a preferred embodiment, the sample is obtained using a device described in WO 2010/085841 which claims priority from US 61/147,718 (Genetic Technologies Ltd, Australia) (referred to herein as the "RareCellect™ device"). Briefly, this device comprises
- an elongate insertion tube having a first end adapted for insertion through the external orifice (external os) of said patient's cervix and a second end including a handle means for manipulating said tube.
- a measuring means for determining the position of said first end of said tube within said cervix
wherein said first end includes a sampling head adapted to collect, biological material including extracellular nucleic acids and mucus wherein said measuring means is adapted to determine the extent of insertion and transcervical position of said first end to optimise the transcervical sampling site.

The measuring means may include a physical stop, a physical scale and/or an ultrasound or similar means for determining the *in situ* position of said head.

The sampling head is preferably adapted to collect or capture a quantity of biological material available from within the patient's cervix including mucous, blood, tissue, DNA and cells. The capture preferably includes adsorption of mucus onto the surface of said head but may include absorption of more liquid sample onto the head.

The sampling head preferably includes a compliant, soft (may not be soft initially) and absorptive sponge-like material adapted to retain a soft feel in the compressed pre-absorptive state and may be chosen from a range of materials including polyvinyl acetate (PVA), polyurethane (PU) and cellulose.

The sampling head may also optionally be formed of hydrophilic materials and the outer surface of the sampling head, may be shielded with the temporary cover or chemical coating. The shield allows the user to selectively control the point at which the sampling head is exposed and commences collecting biological material, including the ability of the temporary cover or coating to protect the sampling head during insertion such that the temporary cover or coating can be removed only when the sampling head is correctly positioned within the cervix.

The shield may be a dissolvable chemical coating or membrane applied to the outer surface of the head, where the chemical coating or membrane is adapted to dissolve over a finite period of time, allowing the sampling head to be inserted without risk of contamination by collecting biological material during the insertion process.

As an alternative, the shield may take the form of a physical barrier of an outer sleeve which is adapted to cooperate coaxially with the insertion tube such that the insertion tube is inserted telescopically within the outer sleeve and the relative movement of the sampling head (and associated insertion tube) with the outer sleeve provides for movement between a first retracted position where the sampling head is fully contained within and protected within the confines of the outer sleeve and a second extended position where the sampling head has been telescopically moved out and away from the outer sleeve and is then exposed for sample collection.

As another alternative, the "shield" may be effected by the pre-use compression of the sponge-like head so as to delay sample collection until the sample head has expanded *in situ.*

The sampling device preferably includes a stop means which can take the form of a first stop adapted to cooperate with the outer sleeve so as to provide a setting means or stop to allow the user to judge the extent of insertion of the sleeve into the patient's cervix. The first stop would be adapted to gently abut the exterior os of the patient's cervix, thereby allowing the patient to carefully judge the extent of insertion of the outer sleeve into the interior of the patient's cervix.

The insertion tube may also include its own stop in the form of a second stop to allow control of the telescopic movement and extension of the sampling head, relative to the outer sleeve.

The sampling head is preferably adapted to expand upon absorption of the sample and expand from a dry compressed state to a swollen state having absorbed and holding the sample.

The sampling head is most preferably configured to provide a high surface area to volume ratio in order to maximise the adsorption of mucus onto the surface of the head. In another embodiment, the head adopts a radially expanded swollen state where the sampling head preferentially expands in a radial direction upon contact with the sampling site and absorption of the sample. The sample head in the expanded swollen state is preferably configured and dimensioned to allow a retraction back into the physical outer sleeve for withdrawal from the patient without substantial loss of absorbed sample.

The absorptive materials in the sample head preferably include a pore size between 10 to 2000 microns with an average pore opening between 400 to 1000 microns. The pore size is preferably configured so that it does not diminish toward the outer surface of the sample head in order to ensure that absorption and in particular adsorption of the sample head is not blocked or constrained by the initial absorption from the surface of the sample head.

Most preferably, the sample head is shaped such that the surface area to volume ratio is the same as, or increased, compared to that of a cylindrical shaped sample head. The sampling head is preferably configured as a multifilamentous array of sponge or sponge-like fingers. The sample head may alternatively be shaped as a cylinder with the cylinder optionally including a hollow for the addition of an optional aspirating means. The preferential overall size of the sampling head in its compressed form, being about 3mm in diameter and allowing an expansion between 5 to 20mm once the predetermined quantity of biological material has been collected.

The sampling head preferably has a length between 1 to 5cm prior to collecting said sample and an expanded volume at the predetermined optimal volume between .5 to 3 cubic centimetres. The expansion is preferably radial.

The predetermined quantity of biological material absorbed or adsorbed by the sampling head is preferably between 0.1 ml and 3 ml but will depend on the nature and condition of the patient.

The insertion tube of the sampling device preferably includes markings along the length thereof adapted to cooperate with the stop means and provide a quantitative measure for the extent of insertion and ultimately the transcervical position of said sampling head once the device is fitted. The outer sleeve when fitted to the device is preferably provided with markings along the length thereof to cooperate with a second stop and provided quantitative measure of the extent of insertion and the transcervical position of the sampling head.

In addition, the elongate tube and/or the sampling head may include an ultrasound readable marker to assist in tracing the inserted position of the sampling head within the patient using ultrasound monitoring.

The outer sleeve, if incorporated into the sampling device may also include an ultrasound readable marker to assist in tracing the position of the sampling head relative to the outer sleeve within the patient.

The sampling device may optionally include an aspirating means including a vacuum generating means. The sampling device when incorporating the optional aspirating means may integrate same with the outer sleeve component thereof which can be adapted to transfer the vacuum as applied to the device to the sampling head.

The sampling device may optionally include a sample storage means and/or transport means to allow the sample to be preserved and stored in a suitable manner to allow transport without the need for transferral thereby maximising preservation of the sample.

Once obtained, the sample is preferably stored at 0 to 4°C until use. The sample is preferably transported and/or stored in HypoThermosol-FRS (HTS-FRS) Medium (Biolife Solutions) at 4°C. For long term storage, the sample is preferably stored in CryoStor CS5(Biolife Solutions) at -80°C.

In a further embodiment, the sample is transported and/or stored in Gibco^{™} AmnioMaxII, Gibco^{™} AmnioMax C-100, or Gibco^{™} Keratinocyte-SFM supplemented with 2% fetal bovine serum, heparin (2500U), hydrocortisone (5 µg/ml), insulin (5 µg/ml), human epidermal growth factor (5µg/ml), human basic fibroblast growth factor (5µg/ml), 25 µg/ml gentamycin, 50 ng/ml amphotericin B, 1-2 mmol/L vitamin C (ascorbic acid) or a water soluble analogue of vitamin E (1 mmol/L Trolox).

In one embodiment, the transport and/or storage media comprises serum such as bovine calf serum or human serum.

In a further embodiment, the storage medium is degassed with nitrogen to reduce oxidative stress to the samples.

The methods of the invention can be performed on any pregnant female of any mammalian species. Preferred mammals include, but are not limited to, humans, livestock animals such as sheep, cattle and horses, as well as companion animals such as cats and dogs. Preferably, the mammal is a human.

The sample may be obtained at any stage of pregnancy. Preferably the sample is obtained during the first and second trimester of pregnancy. More preferably, the sample is obtained in the first trimester of pregnancy. Ideally, the sample is obtained at a stage when a decision can be made for the well-being of the fetus and preferably within a period where an opportunity to make an early decision regarding therapeutic abortion can be made. Preferably, the sample is obtained up to 18 weeks of the pregnancy of a human female.

### Enrichment/Separation of Free Nucleic Acids in the Sample

Using the methods of the invention, free nucleic acids in the sample is enriched/separated from at least 10%, more preferably at least 20%, more preferably at least 30%, more preferably at least 40%, more preferably at least 50%, more preferably 60%, more preferably 70%, more preferably 80%, more preferably 90%, more preferably 95%, more preferably 99%, and most preferably all of the cells and nuclei in the original sample taken from the female. This can be achieved using any method known in the art for separating free nucleic acids from intact cells/nuclei. For example, in one embodiment the sample is centrifuged at about 4,000 to 7,000 g for 5 to 10 minutes, and the supernatant comprising the nucleic acids is aspirated from the pellet comprising cells. This procedure may be repeated to minimise the number of cells in the enriched sample.

The present inventors have found that free fetal nucleic acids obtained using the methods of the invention are larger than the free fetal nucleic acids obtained by other means such as from blood or plasma. As described by Horlitz *et al.* (2009), smaller fragments of DNA, such as less than 300 bp, can lead to an underestimation of the quantity of DNA in a sample. Thus, in a preferred embodiment, at least some of the fetal nucleic acids are greater than about 500 nucleotides in length. More preferably, at least 10%, or at least 25%, or at least 50% of the fetal nucleic acids are greater than about 500 nucleotides in length. In a further embodiment, at least some of the fetal nucleic acids are greater than about 1,000 nucleotides in length.

Methods of DNA extraction are well known in the art. In one embodiment, the is achieved by using a protocol based on extraction using organic solvents such as a mixture of phenol and chloroform, followed by precipitation with ethanol (see, for example, J. Sambrook *et al*., supra). Other methods include: salting out DNA extraction, trimethylammonium bromide salt DNA extraction, and guanidinium thiocyanate DNA extraction. There are also numerous kits that can be used to extract DNA and that are commercially available from, for example, BD Biosciences Clontech (Palo Alto, CA), Epicentre Technologies (Madison, WI), Gentra Systems, Inc. (Minneapolis, MN), MicroProbe Corp. (Bothell, WA), Organon Teknika (Durham, NC), and Qiagen Inc. (Valencia, CA).

The present inventors have surprisingly found that although fetal cells are relatively rare in a cervical sample, the concentration of fetal derived nucleic acids is relatively high. For instance, although there was variation between samples, on average about 10% of the free nucleic acids recovered from the cervical samples was fetal in origin, whereas there are relatively few fetal cells compared to maternal cells. This means that the lysing of maternal cells in the sample is likely to undesirably decrease the fetal nucleic acid concentration. As a result, it is preferred that the sample is handled and processed to minimise the lysis/death of cells.

In another embodiment, the enriched/separated nucleic acids are combined with fetal nucleic acids obtained from the female using other techniques. For example, fetal cells can be enriched from a variety of samples, including the cervical sample, and nucleic acids extracted from these cells. Examples of methods of enriching fetal cells include, but are not limited to, those described in WO 91/016452, WO 95/003431, WO 00/071987, WO 03/020986, WO 03/102595, WO 04/076653, WO 06/120434, WO 06/119569, WO 07/147076 and WO 09/103110.

Whilst free fetal nucleic acids can also be obtained from maternal blood or plasma (see, for example, US 6,258,540 and US 6,664,056), it is typically desirable not to combine these methods with those of the present invention because generally the relative proportion of fetal nucleic acids to maternal nucleic acids is lower in such sample. For example, on average about 10% of the DNA is fetal for the methods of present invention compared to about 3% from maternal plasma by real time polymerase chain reaction (RT-PCR) (see Lo *et al*., 1998b). As another example, on average maternal blood comprise about 25 fetal genome equivalents per ml of blood compared with on average at least about 10,000 fetal genome equivalents per sample using the methods of the invention.

In an embodiment, the nucleic acids can be combined with DNA obtained from an amniotic sample, the isolation of which is described in WO 07/028155.

### Uses

Fetal nucleic acids enriched using the methods of the invention can be analysed for traits of interest and/or abnormalities of the fetus using techniques known in the art. The nucleic acids may encode a protein, or may encode a functional RNA which is not translated, or the DNA analysed may even be an informative non-transcribed sequence or marker.

In one embodiment, chromosomal abnormalities are detected. By "chromosomal abnormality" we include any gross abnormality in a chromosome or the number of chromosomes. For example, this includes detecting trisomy in chromosome 21 which is indicative of Down's syndrome, trisomy 18, trisomy 13, sex chromosomal abnormalities such as Klinefelter syndrome (47, XXY), XYY or Turner's syndrome, chromosome translocations and deletions, a small proportion of Down's syndrome patients have translocation and chromosomal deletion syndromes which include Pradar-Willi syndrome and Angelman syndrome, both of which involve deletions of part of chromosome 15, and the detection of mutations (such as deletions, insertions, transitions, transversions and other mutations) in individual genes. Other types of chromosomal problems also exist such as Fragile X syndrome, hemophilia, spinal muscular dystrophy, myotonic dystrophy, Menkes disease and neurofibromatosis, which can be detected by DNA analysis.

The phrase "genetic abnormality" also refers to a single nucleotide substitution, deletion, insertion, micro-deletion, micro-insertion, short deletion, short insertion, multinucleotide substitution, and abnormal DNA methylation and loss of imprint (LOI). Such a genetic abnormality can be related to an inherited genetic disease such as a single-gene disorder (e.g., cystic fibrosis, Canavan, Tay-Sachs disease, Gaucher disease, Familial Dysautonomia, Niemann-Pick disease, Fanconi anemia, Ataxia telengectasia, Bloom syndrome, Familial Mediterranean fever (FMF), X-linked spondyloepiphyseal dysplasia tarda, factor XI), an imprinting disorder [e.g., Angelman Syndrome, Prader-Willi Syndrome, Beckwith-Wiedemann syndrome, Myoclonus-dystonia syndrome (MDS)], or to predisposition to various diseases (e.g., mutations in the BRCA1 and BRCA2 genes). Other genetic disorders which can be detected by DNA analysis are known such as thalassaemia, Duchenne muscular dystrophy, connexin 26, congenital adrenal hypoplasia, X-linked hydrocephalus, ornithine transcarbamylase deficiency, Huntington's disease, mitochondrial disorder, mucopolysaccharidosis I or IV, Norrie's disease, Rett syndrome, Smith-Lemli Optiz syndrome, 21-hydroxylase deficiency or holocarboxylase synthetase deficiency, diastrophic dysplasia, galactosialidosis, gangliosidosis, hereditary sensory neuropathy, hypogammaglobulinaemia, hypophosphatasia, Leigh's syndrome, aspartylglucosaminuria, metachromatic leukodystrophy Wilson's disease, steroid sulfatase deficiency, X-linked adrenoleukodystrophy, phosphorylase kinase deficiency (Type VI glycogen storage disease) and debranching enzyme deficiency (Type III glycogen storage disease). These and other genetic diseases are mentioned in The Metabolic and Molecular Basis of Inherited Disease, 8th Edition, Volumes I, II, III and IV, Scriver, C. R. et al. (eds), McGraw Hill, 2001. Clearly, any genetic disease where the gene has been cloned and mutations detected can be analysed.

The methods of the present invention can also be used to determine the sex of the fetus. For example, staining of the enriched fetal DNA with a Y-chromosome specific marker will indicate that the fetus is male.

In yet another use of the invention, the methods described herein can be used for paternity testing. Where the paternity of a child is disputed, the procedures of the invention enable this issue to be resolved early on during pregnancy. Many procedures have been described for parentage testing which rely on the analysis of suitable polymorphic markers. As used herein, the phrase "polymorphic markers" refers to any nucleic acid change (e.g., substitution, deletion, insertion, inversion), variable number of tandem repeats (VNTR), short tandem repeats (STR), minisatellite variant repeats (MVR) and the like. Typically, parentage testing involves DNA fingerprinting targeting informative repeat regions, or the analysis of highly polymorphic regions of the genome such as HLA loci.

### Analysis of Fetal Nucleic Acids

Fetal nucleic acids enriched using the methods of the invention can be analysed by a variety of procedures, however, typically genetic assays will be performed. Genetic assay methods include the standard techniques of analysis of methylation patterns, restriction fragment length polymorphism assays, sequencing and PCR-based assays (including multiplex F-PCR STR analysis, whole genome amplification. RT-PCR, digital PCR, and microarray analysis), as well as other methods described below.

The genetic assays may involve any suitable method for identifying mutations or polymorphisms, such as: sequencing of the nucleic acids at one or more of the relevant positions; differential hybridisation of an oligonucleotide probe designed to hybridise at the relevant positions of either the wild-type or mutant sequence; denaturing gel electrophoresis following digestion with an appropriate restriction enzyme, preferably following amplification of the relevant DNA regions; S1 nuclease sequence analysis; non-denaturing gel electrophoresis, preferably following amplification of the relevant DNA regions; conventional RFLP (restriction fragment length polymorphism) assays; selective DNA amplification using oligonucleotides which are matched for the wild-type sequence and unmatched for the mutant sequence or vice versa; or the selective introduction of a restriction site using a PCR (or similar) primer matched for the wild-type or mutant genotype, followed by a restriction digest. The assay may be indirect, ie capable of detecting a mutation at another position or gene which is known to be linked to one or more of the mutant positions. The probes and primers may be fragments of DNA isolated from nature or may be synthetic.

A non-denaturing gel may be used to detect differing lengths of fragments resulting from digestion with an appropriate restriction enzyme. The DNA is usually amplified before digestion, for example using the polymerase chain reaction (PCR) method and modifications thereof.

Compared to at least some other sources of free fetal nucleic acids such as maternal blood or plasma, the average size of free nucleic acids found in cervical samples are relatively large making them more amenable to procedures such as nucleic acid amplification. Amplification of nucleic acids may be achieved by the established PCR methods or by developments thereof or alternatives such as quantitative PCR, quantitative fluorescent PCR (QF-PCR), multiplex ligation dependent probe amplification, digital PCR, RT-PCR, restriction fragment length polymorphism PCR (PCR-RFLP), PCR-RFLP/RT-PCR-RFLP, hot start PCR, nested PCR, *in situ* polonony PCR, *in situ* rolling circle amplification (RCA), bridge PCR, picotiter PCR and emulsion PCR. Other suitable amplification methods include the ligase chain reaction (LCR), transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid based sequence amplification (NABSA). Other amplification methods that can be used herein include those described in US 5,242,794; 5,494,810; 4,988,617; and 6,582,938.

An "appropriate restriction enzyme" is one which will recognise and cut the wild-type sequence and not the mutated sequence or *vice versa.* The sequence which is recognised and cut by the restriction enzyme (or not, as the case may be) can be present as a consequence of the mutation or it can be introduced into the normal or mutant allele using mismatched oligonucleotides in the PCR reaction. It is convenient if the enzyme cuts DNA only infrequently, in other words if it recognises a sequence which occurs only rarely.

In another method, a pair of PCR primers are used which hybridise to either the wild-type genotype or the mutant genotype but not both. Whether amplified DNA is produced will then indicate the wild-type or mutant genotype (and hence phenotype).

A preferable method employs similar PCR primers but, as well as hybridising to only one of the wild-type or mutant sequences, they introduce a restriction site which is not otherwise there in either the wild-type or mutant sequences.

In order to facilitate subsequent cloning of amplified sequences, primers may have restriction enzyme sites appended to their 5' ends. Thus, all nucleotides of the primers are derived from the gene sequence of interest or sequences adjacent to that gene except the few nucleotides necessary to form a restriction enzyme site. Such enzymes and sites are well known in the art. The primers themselves can be synthesized using techniques which are well known in the art. Generally, the primers can be made using synthesizing machines which are commercially available.

PCR techniques that utilize fluorescent dyes may also be used to detect genetic defects in fetal nucleic acids enriched by the methods of the invention. These include, but are not limited to, the following five techniques.
i) Fluorescent dyes can be used to detect specific PCR amplified double stranded DNA product (e.g. ethidium bromide, or SYBR Green I).
ii) The 5' nuclease (TaqMan) assay can be used which utilizes a specially constructed primer whose fluorescence is quenched until it is released by the nuclease activity of the Taq DNA polymerase during extension of the PCR product.
iii) Assays based on Molecular Beacon technology can be used which rely on a specially constructed oligonucleotide that when self-hybridized quenches fluorescence (fluorescent dye and quencher molecule are adjacent). Upon hybridization to a specific amplified PCR product, fluorescence is increased due to separation of the quencher from the fluorescent molecule.
iv) Assays based on Amplifluor (Intergen) technology can be used which utilize specially prepared primers, where again fluorescence is quenched due to self-hybridization. In this case, fluorescence is released during PCR amplification by extension through the primer sequence, which results in the separation of fluorescent and quencher molecules.
v) Assays that rely on an increase in fluorescence resonance energy transfer can be used which utilize two specially designed adjacent primers, which have different fluorochromes on their ends. When these primers anneal to a specific PCR amplified product, the two fluorochromes are brought together. The excitation of one fluorochrome results in an increase in fluorescence of the other fluorochrome.

Fetal nucleic acids enriched using the methods of the invention can also be analysed using the MassARRAY® and SEQureDx^{™} procedures of Sequenom Technology (San Deigo, California, USA), as well as using the methods described in WO 2007/103910 and WO 2009/030100.

### EXAMPLES

### Example 1- Sample Collection

Prior to sampling, the length of the patient's cervical canal was determined by ultrasound or using the following published estimates:

| Gestational age (wks) | Estimated cervical length (cm) |
|---|---|
| 6 | 2-3 |
| 7 | 3 |
| 8 | 3-4 |
| 9 | 4 |
| 10 | 4 |
| 12 | 4-4.5 |

A speculum was used to aid in the collection of the sample. The speculum was carefully inserted into the vagina, letting the speculum follow the path of least resistance. The speculum was opened thereby making the cervix visible to the operator.

Cervical mucus samples (transcervical samples) were collected with one of the three different devices listed below:
(i) Pap smear cytology brush (Medico EndoScan plus) (n=24),
(ii) RareCellect™ biological sampling device described in WO 2010/085841 (Genetic Technologies Ltd, Australia) (n= 47), and
(iii) Aspiracath (Cook Australia) (n=32).

A total of one hundred and three samples were collected from patients undergoing elective termination of pregnancy (TOP) during first trimester of pregnancy with a mean gestational age of eight weeks gestation (range 6-12 weeks). Cellular material and/or free nuclei were removed from the sample by centrifugation.

### Pap Smear Cytology Brush

The cytology brush was inserted to the level of the external os and rotated 360 degrees to collect the specimen. The cytology brush was removed and the brush containing the sample was cut and transferred into a 15ml FALCON™ tube containing transport media or D-PBS. The samples are stored at 4°C ready for transport.

### RareCellect™ Device

The short silicone collar is adjusted so that the distance between the tip of the sheath and the top of the collar is the same, or up to 3mm shorter than the length of the patient's cervical canal. The device was then inserted into the cervical canal until the adjusted collar contacts the external os. Once the device was in position, the plunger was pushed into the sheath, and the sponge was released out the other end.

The device was left in place for up to 2 minutes allowing the sponge to be completely saturated, collecting 0.1-3ml of a sample. The device was carefully removed from the cervical canal. Using the break point, approximately halfway along the handle the device was broken and removed from the outer sheath. The tip of the sampling device with the sponge was transferred into a 15ml FALCON™ tube containing transport media or D-PBS, making sure the sponge is entirely immersed in the media. The samples were stored at 4°C ready for transport.

### Aspiracath

The aspiration catheter was inserted approximately 2-3cm into the endocervical canal at the level of the internal os. A 0.1-1ml sample was collected by gentle aspiration. The catheter was removed and the end of the device containing the sample was cut and transferred into a 15ml FALCON™ tube containing transport media or D-PBS. The samples were stored at 4°C ready for transport.

### Example 2 - Preparation of Cell Free DNA from Supernatant Samples

The sampling device was removed from the transport container using sterile forces and transferred into an organ culture dish containing 1 ml D-PBS.

### Pap Smear Cytology Brush

The mucus was carefully removed from the brush using sterile forceps. The sample was manually tweezed apart using sterile forceps into small pieces. Gentle pipetting using a 1ml pipette further disaggregates the sample. The entire sample was then passed through a cell strainer (100µm Nylon strainer, Becton Dickinson, USA) into a sterile 50ml FALCON™ tube. The brush was then transferred back into the 15ml FALCON™ tube where it was washed with the original 4ml of transport media or D-PBS present in the transport container. The brush was washed by gently agitation, swirling the 15ml FALCON™ tube up and down a few times.

The entire sample was then passed through the same cell strainer into the sterile 50ml FALCON™ tube. A further 4ml D-PBS wash was sometimes required to further remove the mucus/cells from the sponge in the 15ml FALCON™ tube. This step maybe repeated a few times until the brush is completely washed, or there is no evidence of mucus or cells being present on the brush. A total of 12-16ml of sample was collected which comprises of cells and D-PBS media.

### RareCellect™ Device

The sponge was carefully removed from the handle mop using sterile forceps and placed back into the 15ml FALCON™ tube. The sponge was washed with the original 4ml of transport media or D-PBS present in the transport container. The sponge was washed by gently agitation, swirling the 15ml FALCON™ tube up & down a few times. The entire sample was then passed through a cell strainer (100µm Nylon strainer, Becton Dickinson, USA) into a sterile 50ml FALCON™ tube. A further 4ml D-PBS wash was used to remove the mucus/cells from the sponge in the 15ml FALCON™ tube. This step maybe repeated a few times until the sponge is completely washed, or there is no evidence of mucus or cells being present on the sponge. Complete removal sometimes required manual assistance using sterile forceps. A total of 16-20ml of sample was collected which comprises of cells and D-PBS media.

### Aspiracath

The aspirette sample was washed twice from the device with 500 µl PBS. Complete removal sometimes requires manual assistance using sterile forceps. The sample was manually tweezed apart using sterile forceps into small pieces. Gentle pipetting using a 1ml pipette further disaggregated the sample. The sample was transferred back into the original 4ml of D-PBS present in the transport container and washed by gently agitation, swirling the 15ml FALCON™ tube up and down a few times. The entire sample was then passed through a cell strainer (100µm Nylon strainer, Becton Dickinson, USA) into a sterile 50ml FALCON™ tube. A total of 5ml of sample is collected which comprises of cells and D-PBS media.

The 50ml FALCON™ tube containing cells <100µm in size (collected with the Pap Smear cytology brush, RareCellect™ device or Aspiracath) was centrifuged at 2000 rpm for 10 minutes and the supernatant transferred to a new sterile 50ml FALCON™ tube making sure not to disturb the cell pellet. The 50ml FALCON™ tube containing the supernatant was centrifuged at 2000 rpm for 10 minutes and the supernatant transferred to a new 50ml FALCON™ tube. A final centrifugation step at high speed (i.e. 4500G for 10 minutes) was required to remove any cellular material that may still be present in the supernatant sample. The cell free DNA was then collected and stored at -20°C.

### Example 3 - DNA Extraction of Cell Free from Supernatant Samples

The cell free DNA from the supernatant collected in Example 2 was extracted using the Qiagen DNA Blood Maxi kit (Qiagen,USA). The DNA extraction process involved equilibrating the samples to room temperature (RT). Once the samples reached RT, 500µl of Qiagen protease per 10ml sample, 12ml of buffer AL is also added to the sample and mixed vigorously. The sample is incubated at 70°C for 10 minutes.

Prior to transferring the lysate to the column 10ml 100% ethanol was added to the lysate. The entire lysate is transferred to the maxi column and centrifuged at 3000 rpm for 3 minutes (note: this may require multiple lysate to column spins). The maxi column was then washed with 5mL of Buffer AW1 and centrifuged at 5000 rpm for 1 min. Followed by a further 5mL wash with buffer AW2 and centrifuged at 5000 rpm for 15 min. AE buffer was then used to elute the sample off the column. Approximately, 600µL Buffer AE was added to the maxi column and incubated at RT for 5 minutes. The column was centrifuged at 5000 rpm for 2 minutes. The eluate was then collected and transferred back through the column to maximize the DNA yield. The column was centrifuged at 5000 rpm at 2 minutes and the eluate transferred to a sterile 1.5ml tube. The concentration of the DNA was measured using a Nanodrop spectrophotometer.

### Results

The average total cell free DNA collected per sample was 4,393+1,035 (SEM) for the Pap Smear Cytology Brush samples, 3,081+314 (SEM) for the RareCellect™ Device samples and 10,642+2,871 (SEM) for the Aspiracath samples (Table 1).

**Table 1. DNA amount of cell free DNA in supernatant of cervical samples**

| **Sample ID Number** | **Sampling Device** | **Concentration of cell free DNA in supernantant (ng/µl)** | **Total Amount/ sample (ng)** |
|---|---|---|---|
| DH911 | Pap Smear Cytology Brush | 3.3 | 1980 |
| DH912 | Pap Smear Cytology Brush | 4.6 | 2760 |
| DH913 | Pap Smear Cytology Brush | 1.6 | 960 |
| DH914 | Pap Smear Cytology Brush | 1.5 | 900 |
| DH915 | Pap Smear Cytology Brush | 6.2 | 3720 |
| DH916 | Pap Smear Cytology Brush | 4.3 | 2580 |
| DH917 | Pap Smear Cytology Brush | 2.33 | 1398 |
| DH918 | Pap Smear Cytology Brush | 2.82 | 1692 |
| DH919 | Pap Smear Cytology Brush | 2.46 | 1476 |
| DH921 | Pap Smear Cytology Brush | 0.75 | 450 |
| DH922 | Pap Smear Cytology Brush | 14.4 | 24480 |
| DH923 | Pap Smear Cytology Brush | 4.4 | 4400 |
| DH925 | Pap Smear Cytology Brush | 8.2 | 8200 |
| DH926 | Pap Smear Cytology Brush | 9.8 | 9800 |
| DH927 | Pap Smear Cytology Brush | 4.5 | 4500 |
| DH931 | Pap Smear Cytology Brush | 5 | 5000 |
| DH932 | Pap Smear Cytology Brush | 4.7 | 4700 |
| DH933 | Pap Smear Cytology Brush | 6.5 | 6500 |
| DH934 | Pap Smear Cytology Brush | 3.9 | 3900 |
| DH936 | Pap Smear Cytology Brush | 4.7 | 4700 |
| DH937 | Pap Smear Cytology Brush | 1.6 | 960 |
| DH939 | Pap Smear Cytology Brush | 1 | 600 |
| DH940 | Pap Smear Cytology Brush | 15.2 | 9120 |
| DH941 | Pap Smear Cytology Brush | 1.1 | 660 |
| | | | |
| DH885 | RareCellect™ Device | 5.2 | 3120 |
| DH886 | RareCellect™ Device | 1.8 | 1080 |
| DH887 | RareCellect™ Device | 2.3 | 1380 |
| DH888 | RareCellect™ Device | 1.6 | 960 |
| DH889 | RareCellect™ Device | 5.5 | 3300 |
| DH890 | RareCellect™ Device | 1.7 | 1020 |
| DH891 | RareCellect™ Device | 3.8 | 2280 |
| DH892 | RareCellect™ e Device | 2.3 | 1380 |
| DH893 | RareCellect™ Device | 3.1 | 1860 |
| DH894 | RareCellect™ Device | 1.7 | 1020 |
| DH895 | RareCellect™ Device | 2.7 | 1620 |
| DH896 | RareCellect™ Device | 2.4 | 1440 |
| DH897 | RareCellect™ Device | 4.4 | 2640 |
| DH898 | RareCellect™ Device | 7.2 | 4320 |
| DH899 | RareCellect™ Device | 6.8 | 4080 |
| DH900 | RareCellect™ Device | 4.2 | 2520 |
| DH901 | RareCellect™ Device | 17.3 | 10380 |
| DH902 | RareCellect™ Device | 9.2 | 5520 |
| DH903 | RareCellect™ Device | 9 | 5400 |
| DH906 | RareCellect™ Device | 1.6 | 960 |
| DH908 | RareCellect™ Device | 8.1 | 4860 |
| DH909 | RareCellect™ Device | 2.7 | 1620 |
| DH910 | RareCellect™ Device | 5.6 | 3360 |
| DH911 | RareCellect™ Device | 1.8 | 1080 |
| DH912 | RareCellect™ Device | 3.9 | 2340 |
| DH913 | RareCellect™ Device | 1.5 | 900 |
| DH914 | RareCellect™ Device | 1.7 | 1020 |
| DH915 | RareCellect™ Device | 9.6 | 5760 |
| DH916 | RareCellect™ Device | 3.4 | 2040 |
| DH917 | RareCellect™ Device | 2.05 | 1230 |
| DH918 | RareCellect™ Device | 2.8 | 1674 |
| DH919 | RareCellect™ Device | 9.2 | 5502 |
| DH921 | RareCellect™ Device | 1.8 | 1080 |
| DH922 | RareCellect™ Device | 10.5 | 6300 |
| DH923 | RareCellect™ Device | 4.8 | 2880 |
| DH925 | RareCellect™ Device | 13.1 | 7860 |
| DH926 | RareCellect™ Device | 8.1 | 4860 |
| DH927 | RareCellect™ Device | 5.8 | 5800 |
| DH931 | RareCellect™ Device | 2.7 | 2700 |
| DH932 | RareCellect™ Device | 3.8 | 3800 |
| DH933 | RareCellect™ Device | 5.7 | 5700 |
| DH934 | RareCellect™ Device | 5.5 | 5500 |
| DH936 | RareCellect™ Device | 3.1 | 3100 |
| DH937 | RareCellect™ Device | 2.4 | 1440 |
| DH939 | RareCellect™ Device | 0.7 | 420 |
| DH940 | RareCellect™ Device | 6.2 | 3720 |
| DH941 | RareCellect™ Device | 3.3 | 1980 |
| | | | |
| DH944 | Aspiracath | 10.8 | 10800 |
| DH945 | Aspiracath | 10.1 | 10100 |
| DH946 | Aspiracath | 3.4 | 3400 |
| DH947 | Aspiracath | 15.5 | 15500 |
| DH948 | Aspiracath | 10.3 | 6180 |
| DH949 | Aspiracath | 3.4 | 2040 |
| DH950 | Aspiracath | 4.6 | 2760 |
| DH951 | Aspiracath | 1.4 | 840 |
| DH952 | Aspiracath | 9.4 | 5640 |
| DH953 | Aspiracath | 4.7 | 2820 |
| DH955 | Aspiracath | 11.6 | 11600 |
| DH956 | Aspiracath | 6.2 | 6200 |
| DH957 | Aspiracath | 28 | 28000 |
| DH958 | Aspiracath | 2.5 | 1500 |
| DH959 | Aspiracath | 36.9 | 22140 |
| DH960 | Aspiracath | 3.2 | 1920 |
| DH969 | Aspiracath | 7.7 | 7700 |
| DH970 | Aspiracath | 16.5 | 16500 |
| DH971 | Aspiracath | 11.1 | 11100 |
| DH972 | Aspiracath | 19.1 | 19100 |
| DH973 | Aspiracath | 4.3 | 4300 |
| DH974 | Aspiracath | 5.1 | 5100 |
| DH979 | Aspiracath | 5.5 | 5500 |
| DH980 | Aspiracath | 5.3 | 5300 |
| DH981 | Aspiracath | 3.7 | 3700 |
| DH982 | Aspiracath | 14.2 | 14200 |
| DH983 | Aspiracath | 2.5 | 2500 |
| DH984 | Aspiracath | 4.7 | 4700 |
| DH985 | Aspiracath | 92.3 | 92300 |
| DH986 | Aspiracath | 4.1 | 4100 |
| DH987 | Aspiracath | 6.9 | 6900 |
| DH988 | Aspiracath | 6.1 | 6100 |

### Example 4 - Quantitative Analysis of Cell Free Fetal DNA in Cervical Samples

All samples were analysed with real time polymerase chain reaction (RT-PCR) for Y-chromosome markers and multiplex fluorescent polymerase chain reaction (MF-PCR) for polymorphic STR markers to provide evidence of fetal DNA within the samples.

### Real Time PCR

The real time PCR analysis was performed using the Stratagene MX3000P thermocycler (Stratagene, La Jolla, CA, USA). Extracted DNA from Example 3 was analysed for DYS 14 and β-globin. The multicopy DYS14 sequence located on TSPY gene was used to measure the quantity of fetal male DNA and the β-globin sequence was used to confirm the presence and quality of total (fetal and maternal DNA) in each sample.

Primers for the DYS14 gene were DYS14 FORWARD and DYS14 REVERSE described by Zhong *et al.* (2006), and primers for the β-globin house keeping gene were β-globin-forward and β-globin-reverse described by Lo *et al.* (1998b).

The singleplex reactions were set up in a total of 25 µl, using 12.5 µl of 1x Brilliant SYBER Green mastermix (Stratagene), 0.25 µl ROX, 10pmole of each primer, and 5 µl of extracted DNA.

The cycling conditions for all reactions consisted of: 15min at 95°C (for sample denaturation and enzyme activation), followed by 50 cycles of 94°C/15 seconds; 60°C/40 seconds; 72°C/15 seconds; 78°C/15 seconds (with plate reads for FAM and Rox at 72°C and 78°C) and melt curve analysis following amplification.

Each sample was performed in triplicate for the DYS 14 reactions and duplicate for the β-globin reactions. Analysis was performed automatically using the MX3000P software with an adaptive baseline set. The number of male or total genomes was derived by comparison with a six point standard curve. Standard curves were generated from 10-fold serial dilutions of reference human genomic male DNA (Promega) ranging in concentration from 10000 genomes to 0.1 genomes per reaction. Levels of cell free DNA were calculated according to Davalieva *et al.* (2006) and expressed in genome equivalents per sample by using 6.6pg per cell as a conversion factor. Samples were identified as Y-positive only if all three reactions were positive, and the melting curve analysis indicated specific amplification.

### MF-PCR Analysis

A total of 47 cell free DNA samples collected with the RareCellect™ prototype device were further tested by multiplex fluorescent polymerase chain reaction (MF-PCR) using four polymorphic short tandem repeat (STR) markers on chromosome 21 (D21S11, D21S1413, D21S1437 and D21S1442).

STR profiles were derived following analysis of the PCR amplified products on 3130 Genetic Analyser (Applied Biosystems) using GeneMapper version 3.7 software. Comparison of the cell free DNA samples and the maternal STR buccal cell profiles confirmed fetal alleles. Samples containing fetal-derived DNA molecules were expected to show three fluorescent peaks for each STR marker allele (a mixed STR profile), two shared with the mother and the other inherited from the father. In the same MF-PCR reaction primers for the polymorphic hypoxanthine guanine phosphoribosyl transferase (HPRT) marker and the amelogenin X and Y gene were also included to simultaneously determine the sex of the fetus. Samples shown to be from male derived fetuses should exhibit full concordance with RT-PCR results. MF-PCR cannot consistently detect paternal alleles containing fetal material less than <10%.

### Results

The results can be summarised as follows:
- Of the 103 transcervical samples tested using three different devices, 13% of the Pap Smear Cytology Brush samples (n=3), 45% of the RareCellect™ Device samples (n=21) and 38% of the Aspirette samples (n=12) exhibited a positive Y-signal by RT-PCR using the DYS14 marker (Table 2).
- The mean percentage of total cell free DNA expressing a Y-signal was 3.6% (range 0.3-10%; median 0.4%), 9.3% (range 0.1-40.1%; median 3.1%), and 11% (range 0.7-59.4%; median 4.8%) for the Pap Smear Cytology Brush, RareCellect™ Device and Aspirette samples, respectively.
- The average number of DYS14 copies per cell free DNA sample was 609 copies (range 140-1,394; median 292), 7,441 copies (range 128-33,684; median 1390) and 14,502 copies (range 1,453 -61,180; median 5596) for the Pap Smear Cytology Brush, RareCellect™ Device and Aspirette samples, respectively.
- Both the Pap Smear Cytology Brush and RareCellect™ Device samples delivered broadly similar total quantities of cell free DNA based the β-globin measurements. The mean total DNA recovered from the Pap Smear Cytology Brush was 118,895 Genome Equivalents per sample (range 3,360-1,177,760; median 44,676) and for the RareCellect™ Device a mean total DNA recovered was 91,853 Genome Equivalents (range 1,558-474,120; median 39,132). While, Aspirette samples had approximately twice the amount of cell free DNA recovered with a mean of 219,988 Genome Equivalents per sample (range 2,790-1,171,000; median 209,000).
- Of the 47 samples analyzed, paternally inherited alleles were observed in 22 cases (~47%) by MF-PCR. All twenty two cell free fetal DNA positive samples showed the presence of two maternal alleles and only one paternal allele (Figure 2).
- There was 100% concordance in sexing (n=22) with RT-PCR data in those samples where paternal alleles were detected by MF-PCR (Table 3).

In summary, sixty four percent of total samples tested using both RT-PCR and MF-PCR showed evidence of fetal derived DNA molecules present in the sample. Of which, nine were shown to be from female derived fetuses and the remaining twenty one from male derived fetuses.

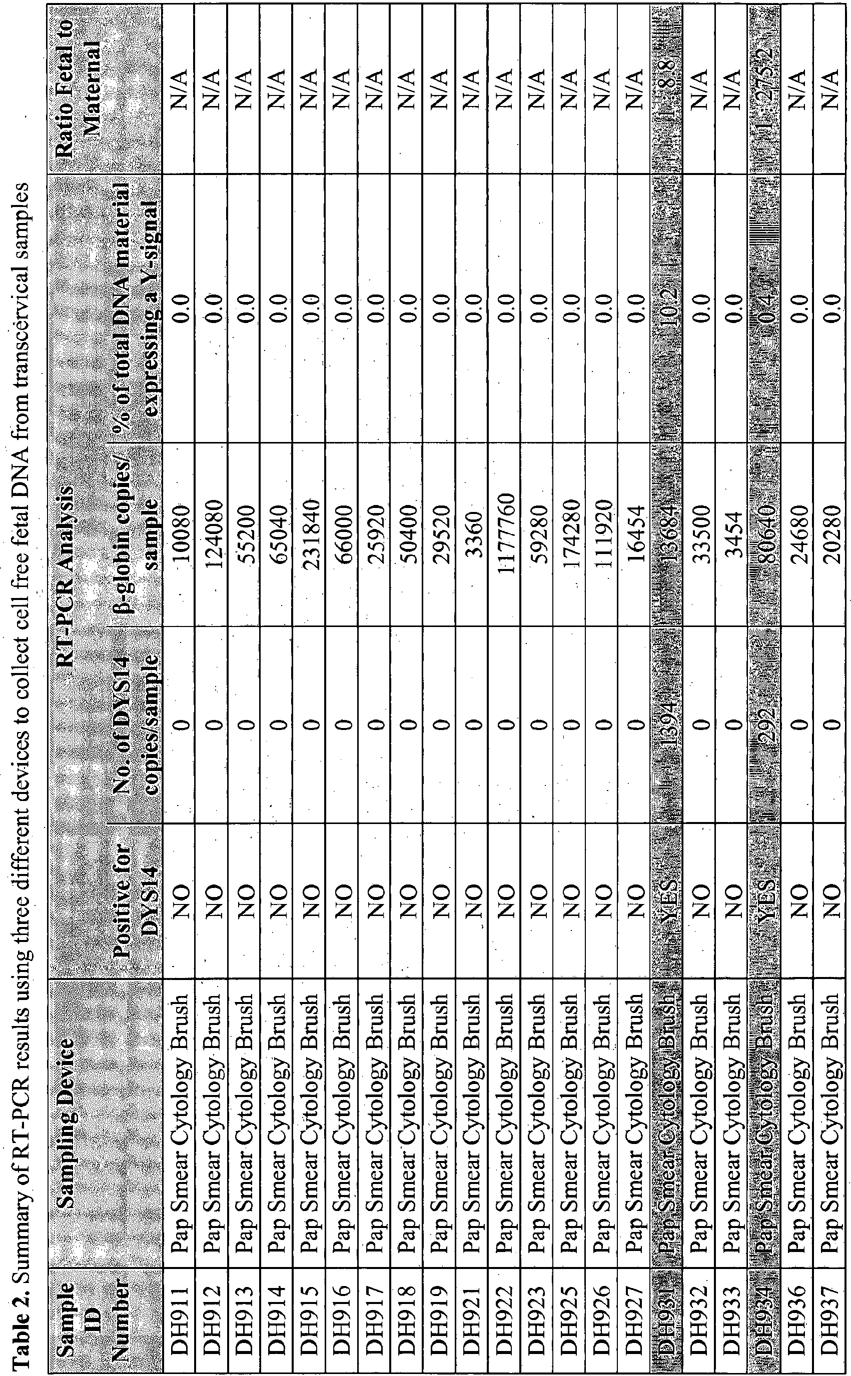

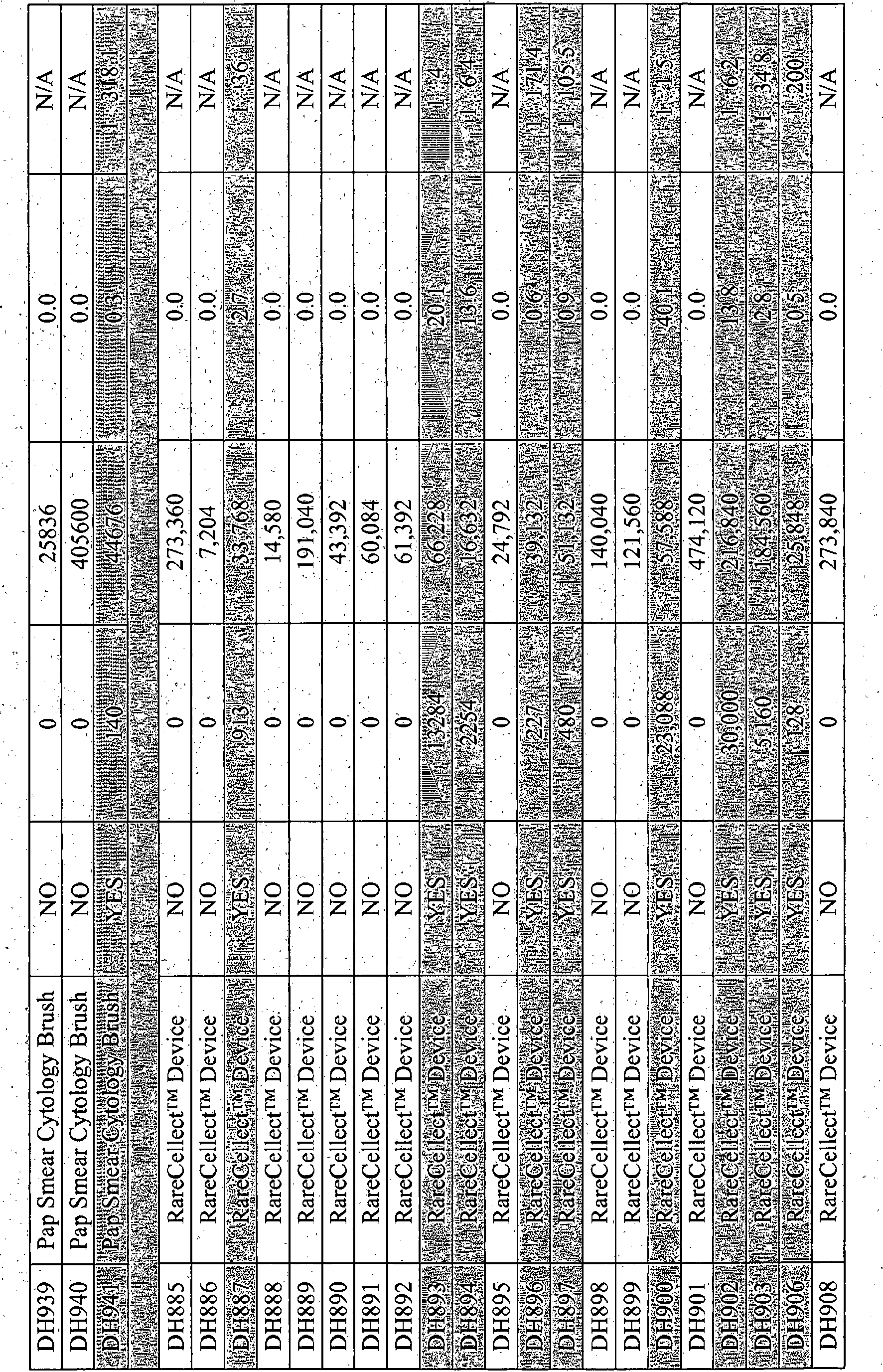

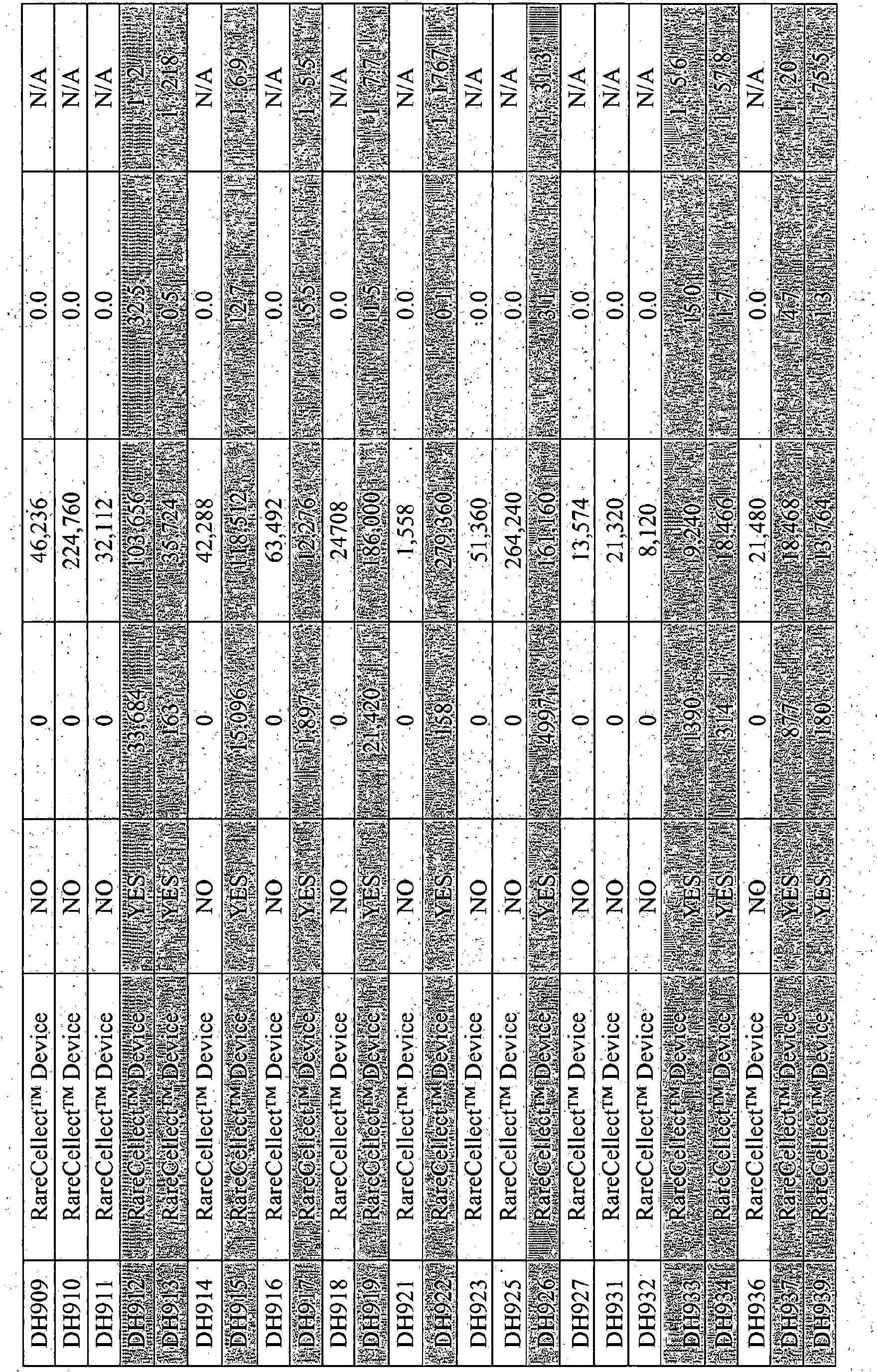

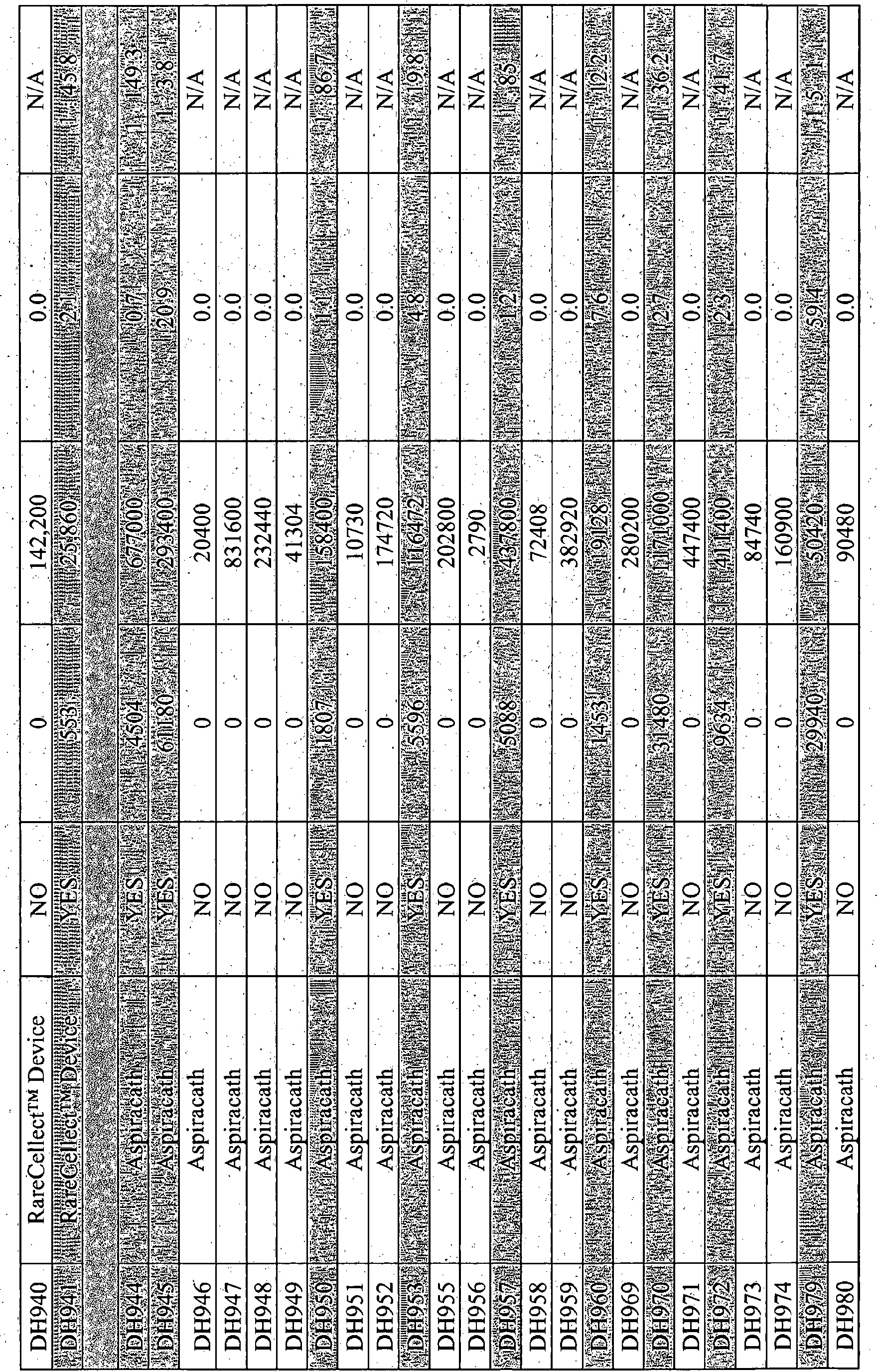

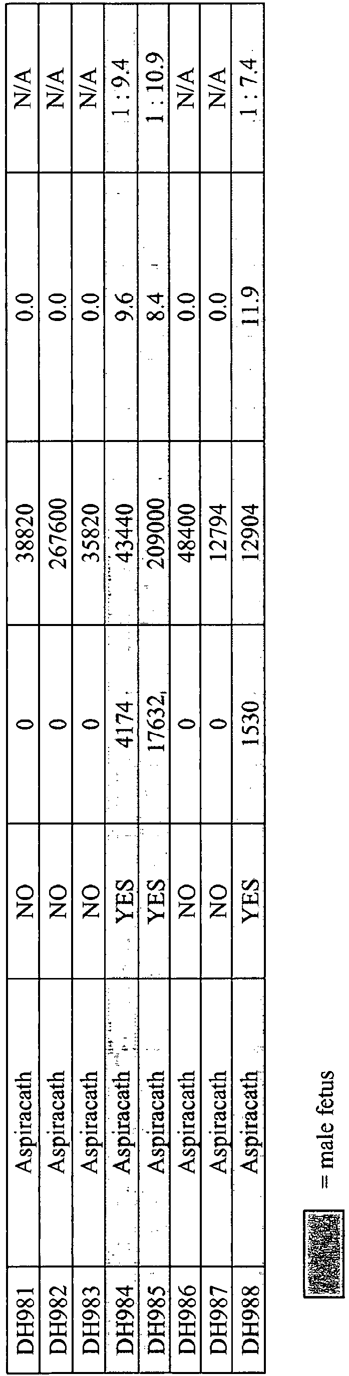

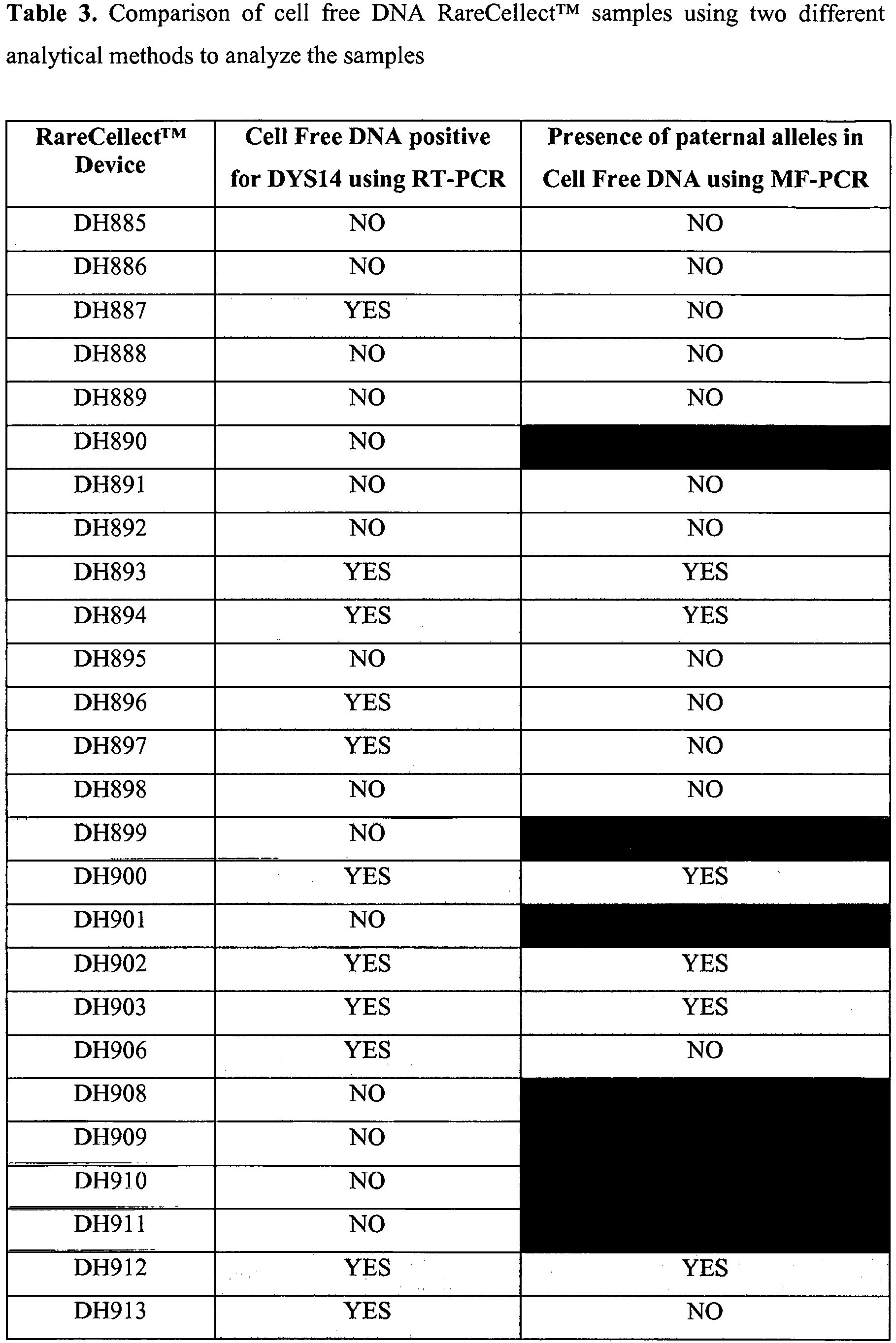

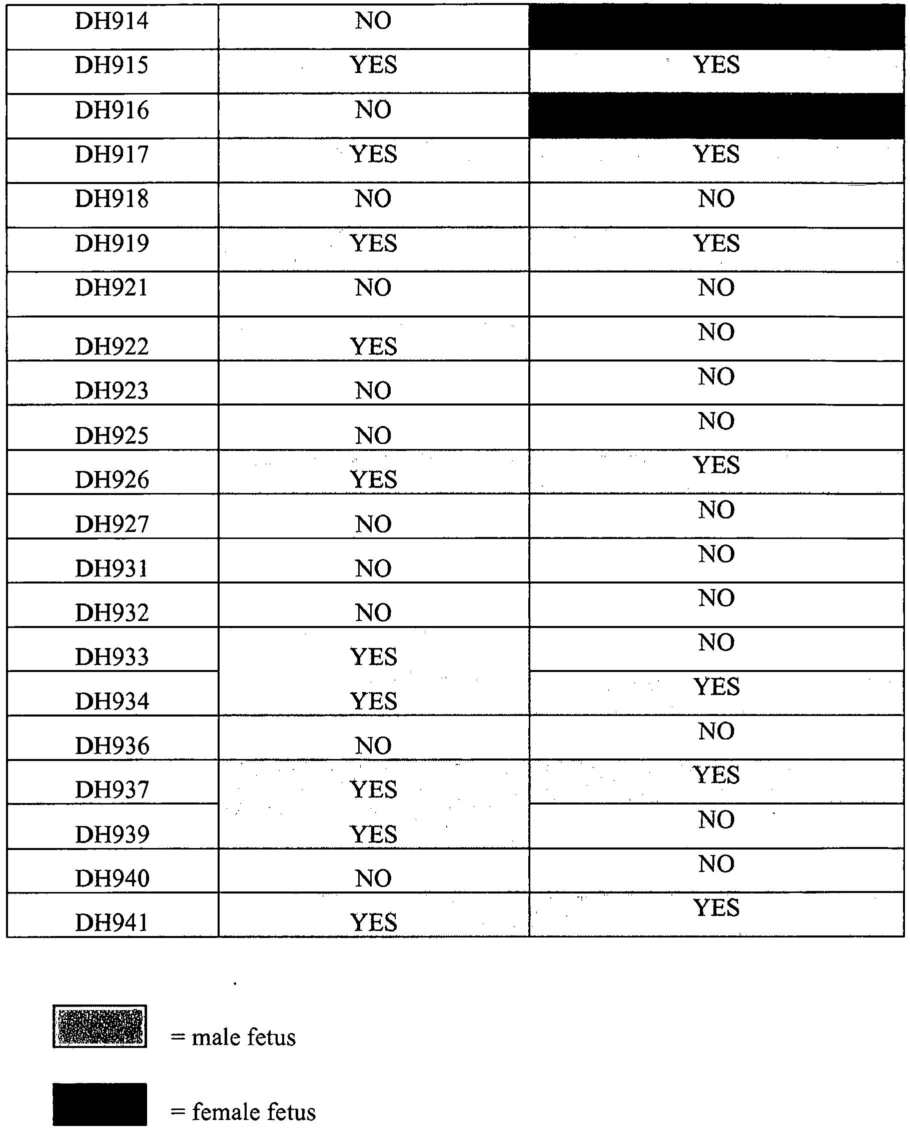

### Example 5 - Size Distribution of Fetal DNA in Recovered Cell Free DNA

A further three samples of cell free DNA recovered from supernatant of aspirette samples were analyzed by gel electrophoresis after Qiagen Maxi DNA extraction to determine the size distribution of circulating fetal DNA in cervical mucus. Size fractionation using gel electrophoresis was then employed to further enrich the fetal material in these samples. Samples were selected on the basis that they were fetal positive but sperm negative by histology.

All three samples had been previously shown by RT-PCR with the DYS14 marker to contain fetal cell free DNA (refer to Example 4). 20µl of cfDNA samples were loaded onto a 2% agarose gel with 10µl of Syber Gold loading buffer. The 2% agarose gel and electrophoresed in 0.5% TBE buffer (45mM Tris-Borate, 1mM EDTA, pH8.0) at 30V for 6hrs. Size distributions of sample cfDNA were estimated by comparison with the 100 base pair DNA ladder. The gel was cut into pieces corresponding to specific DNA sizes according to the DNA size markers. The DNA sizes contained by the specific gel fragments were 100-500 bases, 500-1000 bases, > 1.5kb. The DNA was purified from the agarose gel pieces using the QIAEXII Gel extraction kit (Qiagen) and eluted in 40µl Buffer AE. The samples were subjected to RT-PCR using DYS14 and β-globin marker.

### Results

The results can be summarised as follows:
- Cell free fetal DNA in cervical mucus samples is of high molecular weight (>500 base pairs) (Figure 3).
- By utilizing samples negative for sperm but high in yield for fetal DNA (10%-60%), the recovered cell free DNA which is of high molecular weight is not derived from sperm lysing but from cell free DNA derived from fetal material.
- Size separation using conventional gel electrophoresis (below 300 bases pairs) cannot be utilized to selectively enrich extracellular fetal DNA in the supernatant from cervical samples.
- Most of the fetal DNA was greater 500 bp, with fragments greater than 1kb also being present.

### Example 6 - Diagnostic Utility of Cell Free Fetal DNA from Transcervical Samples

The diagnostic utility of circulatory fetal DNA from cervical mucus samples was demonstrated by screening samples for Cystic Fibrosis (CF) delta 508 mutations. Cell free DNA samples collected with the RareCellect™ device shown to carry fetal DNA by RT-PCR or MF-PCR were only used in this study (see Example 4).

### Cystic Fibrosis Delta 508 mutation screening

Cystic fibrosis is the most common severe autosomal recessive disease in the Caucasian population affecting about one in 2500 births with corresponding carrier frequency of about one in 25 (Boat *et al.,* 1989). The disorder is caused by mutations in the cystic fibrosis transmembrane regulator (CFTR) gene on chromosome 7 (Riordan *et al.,* 1989). When two carriers come together, their offspring have a 25% chance of having CF and a 50% chance of being carriers. Due to the relatively high carrier frequency in Caucasian populations, it represents one of the most common requested tests for prenatal diagnosis of newborns. Although there are multiple mutations which cause CF, the most common accounting for 70-75% of all CF chromosomes, is a specific 3 base pair deletion resulting in the absence of a phenylalanine residue at position 508 in the polypeptide (Δ508). Using ΔF508 as a suitable marker for conducting model testing for CF, the present inventors were able to screen fetal positive cell free DNA samples from cervical mucus.

Fluorescently labelled PCR was used to amplify a 95 base pairs region surrounding the delta 508 mutation (3bp deletion in exon 10) of the CF gene. PCR amplified products were analysed on a 3730 Genetic Analyser (Applied Biosystems) using GeneMapper version 3.7software. Test development of the PCR based assay was performed using commercially obtained male genomic DNA known to be affected with the delta F508 mutation of the CF gene, showing a single fluorescent peak at 92bp. Homozygous wild-type DNA samples (non-affected) were expected to show a single fluorescent peak at 95bp. Heterozygous DNA samples (carriers) were expected to show two fluorescent peaks at 92bp and 95bp (Figure 4). The assay demonstrated that 68pg the equivalent of approximate 10 genomes (10 cells) was sufficient to detect the delta F508 mutation in a mixed sample containing both mutant and wild-type DNA (data not included).

The cycling conditions were 15min at 95°C (for sample denaturation and enzyme activation), 35 cycles of 95°C/15 seconds; 58°C/60 seconds; 72°C/45 seconds; 72°C/5 seconds. 1µl of template DNA (1ng/µl) was used for each reaction.

CF delta F508 affected cell free fetal DNA samples (containing both fetal + maternal carrier DNA) were expected to show both the mutant and wild-type alleles present in the sample, however the proportion of mutant allele was expected to be greater. For example, if the mutant allele was at least twice the size of the wild-type allele then the fetus could be confidently diagnosed as being affected for the CF. It should be noted that at least >5% fetal material in a sample was required to confidently diagnose CF delta F508 in a mixed sample containing both fetal (affected) and maternal DNA (carrier) (data not included).

While, carrier cell free fetal DNA samples could only be identified if the mother was homozygous wild-type for the mutation in a mixed sample. If she also was heterozygous for the delta F508 mutation then an additional marker (such as sex marker or chromosomal 21 STR markers) would be employed in the PCR assay to help differentiate between the mothers DNA and the fetal contribution in the sample.

Matching maternal DNA samples were only screened when cell free fetal DNA samples showed evidence of the mutant allele present in the sample.

Two cell free DNA samples with their matching maternal DNA samples were subjected to additional analysis using multiplex fluorescent PCR with the sex marker, amelogenin and the CF delta F508 marker. Each sample was tested 10 times in a 96-well plate, the ratios of mutant to wild-type were calculated and the status of the fetus for the CF delta F508 mutation was reported.

### Results

The results can be summarised as follows:
- A total of 30 cell free fetal positive DNA RareCellect device samples (n= 21 male; n= 9 female) were screened for the CF delta F508 mutation using fluorescent PCR.
- Of the 30 samples tested, twenty eight were shown to be CF delta F508 homozygous wild-type (non-affected) and two were heterozygous (sample numbers: #DH900 and #DH903) for the delta F508 mutation (Table 4). Ratios of mutant to wild-type alleles for both samples were close to 1:1.
- PCR amplification of matching maternal DNA profiles of samples #DH900 and #DH903 confirmed carrier status of the mothers DNA.
- Additional analysis with the sex marker (amelogenin) and CF delta F508 marker in a multiplex reaction (n=10) allowed sexing of the fetus and

confirmation of the presence of fetal material in both samples thereby allowing the diagnosis of CF.
- In this instance, both samples were shown to contain enriched cell free fetal material from male fetuses (Figure 5). The proportion of fetal material in the two samples varied significantly. Sample #DH900 fetal contribution was ~37% of total sample while, sample #DH903 had limited amount of fetal material corresponding to ~2% of total sample. These results are consistent with the RT-PCR results (see Example 4)
- Correct CF diagnosis for the delta F508 mutation could only be obtained from sample #DH900. The calculated ratios between the mutant and wild-type alleles in multiple reactions showed ratios close to 1:1 thereby confirming the carrier status of the fetus for the CF delta F508 mutation.
- Sample #DH903 was non-informative for the CF delta F508 mutation. This model system for CF diagnosis cannot report on cell free DNA samples where the fetal to maternal contribution is less than 5% in the total sample.

### Example 7 - Conclusions

The present inventors have demonstrated that transcervical sampling using three different sampling devices can deliver cell free fetal DNA in addition to syncytiotrophoblasts, cytotrophoblasts and trophoblast nuclei which can be used for genetic testing.

It is of interest that the RareCellect™ device, which utilizes a PVA sponge to absorb the sample, provides a marked performance enhancement for cell free fetal material when compared to the Pap Smear Cytology Brush device. Whilst both devices appear to give essentially similar overall DNA recoveries, the reason for the enhanced fetal DNA recovery is not immediately clear, however it may be influenced by the site of sampling (i.e. external os v's internal os).

Of the forty-seven samples obtained using the RareCellect™ device and tested by RT-PCR, forty five percent (n=21) showed evidence of the presence of male fetal DNA, providing an estimated 90% total fetal DNA recovery.

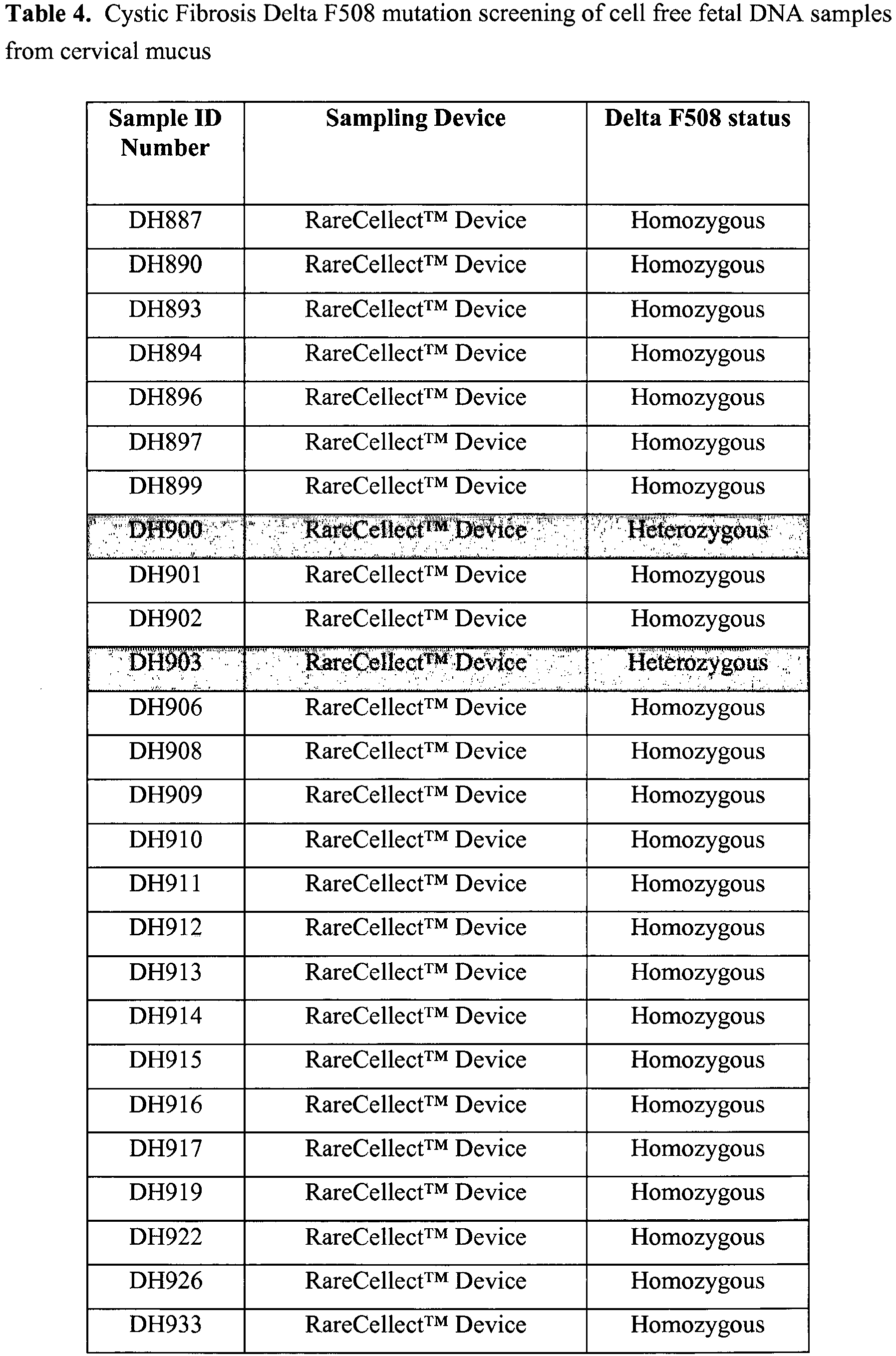

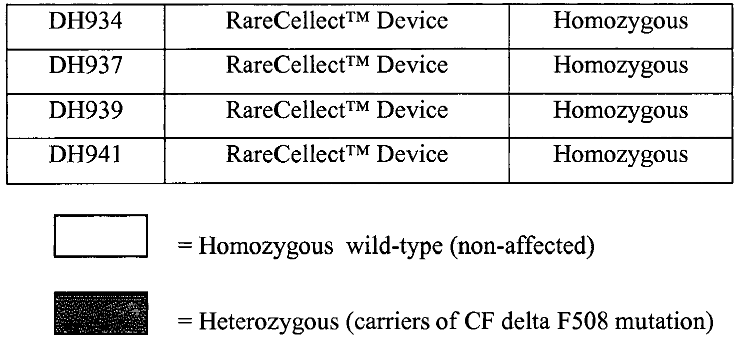

The RareCellect™ device allows the recovery of approximately 7,000 DYS14 Genome Equivalents per sample, which is a much greater fetal DNA contribution than that, reported in the maternal circulation (i.e. 25 Genome Equivalents per/mL of blood). Furthermore, a cell free DNA sample allows the recovery of at least 9.3% cell free fetal material (range 0.1-40%) of total DNA. This is an important observation given that this value is about three times the reported concentration of circulating fetal DNA in healthy maternal plasma in early trimester, which has a mean of 3.4% (range 0.032-11.9%) of the total plasma DNA (Lo *et al,* 1998b). Forty three percent of total cell free DNA samples collected with the RareCellect™ device exhibited concentrations of extracellular fetal DNA greater than 10%. This value is expected to increase with improved sampling efficiency.

It is also clear that the recovered cell free fetal DNA from cervical samples is larger than that previously reported for cell free DNA in both blood and amniotic fluid, which is in the range 50-300 base pairs. The size distribution of the DNA is consistent with recently lysed cellular material and not apoptotic DNA fragmentation.

In addition, the inventors have also shown that size DNA fractionation using gel electrophoresis cannot be employed on extracted cell free DNA from supernatants samples to further enrich fetal DNA.

Although an aspiration device (Aspiracath) was demonstrated to retrieve greater quantities of total sample overall (fetal + maternal), there are known safety problems with aspiration devices, and the results not as consistent and uniform, when compared to the RareCellect™ device.

In addition, the inventors have demonstrated the diagnostic potential of using cell free fetal DNA cervical samples to assess autosomal recessive conditions such as cystic fibrosis.

The present embodiments are to be considered in all respects as illustrative and not restrictive.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

### REFERENCES

Bianchi et al. (2001) Clinical Chemisty 47:1867-9.
Boat et al. The metabolic bases of inherited diseases. In Scriver et al.(eds) The methobolic bases of inherited diseases. 2nd edition McGraw-Hill, New York USA pp. 2649-2680.
Davalieva et al. (2006) J. Matern. Fetal Neonatal. Med. 19: 337-342.
Horlitz et al. (2009) PLoS One 4:e7207.
Holzgreve and Hahn, Bailliere's Clinical Obstetrics and Gynaecology, Vol.14, no. 4, 709-722, 2000
Lee et al. (2002) Am. J. Obstet. Gynecol. 187:1217-21.
Lo et al. (1997) Lancet 350:485-7.
Lo et al. (1998a) N. Engl. J. Med. 339:1734-8.
Lo et al. (1998b) Am. J. Hum. Genet. 62: 768 -775.
Peter et al. (2008) Diagnostic Molecular Pathology 17:185-90.
Riordan et al. (1989). Science. 245:1066-1073.
Wataganara et al. (2003) Hum. Genet. 112:204-8.
Zhong et al. (2006) Prenat. Diagn. 2006. 26:850-854.

## Claims

1. A method of enriching fetal nucleic acids from a pregnant female, the method comprising
i) obtaining a cervical sample, and
ii) enriching extracellular nucleic acids comprising fetal nucleic acids by separating cells and/or cell nuclei from the extracellular nucleic acids in the sample.

2. A method for analysing the genotype of a fetus at a locus of interest, the method comprising
i) obtaining a cervical sample,
ii) separating cells and/or cell nuclei from the extracellular nucleic acids comprising fetal nucleic acids in the sample, and
iii) analysing the fetal nucleic acids for the genotype of a fetus at a locus of interest.

3. The method of claim 3, wherein the nucleic acids is analysed for a genetic abnormality linked to a disease state, or predisposition thereto.

4. A method of determining the sex of a fetus, the method comprising
i) obtaining a cervical sample,
ii) separating cells and/or cell nuclei from the extracellular nucleic acids comprising fetal nucleic acids in the sample, and
iii) analysing the fetal nucleic acids to determine the sex of the fetus.

5. A method of determining the father of a fetus, the method comprising
i) obtaining a cervical sample,
ii) separating cells and/or cell nuclei from the extracellular nucleic acids comprising fetal nucleic acids in the sample,
iii) determining the genotype of the candidate father at one or more loci,
iv) analysing the fetal nucleic acids to determine the genotype of the fetus at one or more of said loci, and
v) comparing the genotypes of iii) and iv) to determine the probability that the candidate father is the biological father of the fetus.

6. The method according to any one of claims 1 to 5, wherein at least some of the fetal nucleic acids are greater than about 500 nucleotides in length.

7. The method according to any one of claims 1 to 6, wherein the cervical sample is a transcervical sample.

8. The method according to any one of claims 1 to 7, wherein the sample is obtained from between the internal os and the external os of the cervix.

9. The method of claim 7 or claim 8, wherein the sample is obtained closer to the internal os of the cervix.

10. The method according to any one of claims 1 to 9, wherein the sample is obtained using a device adapted for transcervical sampling of biological materials from a pregnant female, the device comprising:
- an elongate insertion tube having a first end adapted for insertion through the external orifice (external os) of the cervix and a second end including a handle means for manipulating said tube;
- a measuring means for determining the position of said first end of said tube within the cervix,
wherein said first end includes a sampling head adapted to collect biological material including extracellular nucleic acids and mucus, and wherein said measuring means is adapted to determine the extent of insertion and transcervical position of said first end to optimise the transcervical sampling site.

11. The method according to any one of claims 1 to 10, wherein the cells and/or cell nuclei are separated from the extracellular nucleic acids by centrifugation.

12. The method according to any one of claims 1 to 11, wherein the sample was obtained within 5 to 18 weeks of pregnancy.

13. The method according to any one of claims 1 to 12 which comprises obtaining the sample from the pregnant female.

## Patentansprüche

1. Verfahren zur Anreicherung fetaler Nukleinsäuren von einer schwangeren Frau, wobei das Verfahren Folgendes umfasst
i) Entnehmen einer Gebärmutterhalsprobe, und
ii) Anreichern extrazellulärer Nukleinsäuren, die fetale Nukleinsäuren enthalten, durch Trennen der Zellen und/oder Zellkerne von den extrazellulären Nukleinsäuren in der Probe.

2. Verfahren zur Analyse des Genotyps eines Fetus an einem Locus von Interesse, wobei das Verfahren Folgendes umfasst
i) Entnehmen einer Gebärmutterhalsprobe,
ii) Trennen von Zellen und/oder Zellkernen von den extrazellulären Nukleinsäuren in der Probe, die fetale Nukleinsäuren enthalten, und
iii) Analysieren der fetalen Nukleinsäuren auf den Genotyp eines Fetus an einem Locus von Interesse.

3. Verfahren nach Anspruch 3, wobei die Nukleinsäuren auf eine genetische Anomalität analysiert werden, die mit einem Krankheitsbild oder einer Prädisposition dafür in Verbindung gebracht wird.

4. Verfahren zur Bestimmung des Geschlechts eines Fetus, wobei das Verfahren Folgendes umfasst
i) Entnehmen einer Gebärmutterhalsprobe,
ii) Trennen von Zellen und/oder Zellkernen von den extrazellulären Nukleinsäuren in der Probe, die fetale Nukleinsäuren enthalten, und
iii) Analysieren der fetalen Nukleinsäuren, um das Geschlecht des Fetus zu bestimmen.

5. Verfahren zur Bestimmung des Vaters eines Fetus, wobei das Verfahren Folgendes umfasst
i) Entnehmen einer Gebärmutterhalsprobe,
ii) Trennen von Zellen und/oder Zellkernen von den extrazellulären Nukleinsäuren in der Probe, die fetale Nukleinsäuren enthalten,
iii) Bestimmen des Genotyps des Vaterschaftskandidaten an einem Locus oder mehreren Loci,
iv) Analysieren der fetalen Nukleinsäuren, um den Genotyp des Fetus an einem oder mehreren der Loci zu bestimmen, und
v) Vergleichen der Genotypen aus iii) und iv), um die Wahrscheinlichkeit zu bestimmen, dass der Vaterschaftskandidat der biologische Vater des Fetus ist.

6. Verfahren nach einem der Ansprüche 1 bis 5, wobei wenigstens einige der fetalen Nukleinsäuren mehr als ungefähr 500 Nukleotide lang sind.

7. Verfahren nach einem der Ansprüche 1 bis 6, wobei die Gebärmutterhalsprobe eine transzervikale Probe ist.

8. Verfahren nach einem der Ansprüche 1 bis 7, wobei die Probe zwischen dem inneren und dem äußeren Muttermund des Gebärmutterhalses entnommen wird.

9. Verfahren nach Anspruch 7 oder Anspruch 8, wobei die Probe näher am inneren Muttermund des Gebärmutterhalses entnommen wird.

10. Verfahren nach einem der Ansprüche 1 bis 9, wobei die Probe unter Verwendung einer Vorrichtung entnommen wird, die für die transzervikale Probenentnahme biologischer Materialien aus schwangeren Frauen angepasst ist, wobei die Vorrichtung Folgendes umfasst:
- eine verlängerte Einführröhre, die ein erstes Ende, das angepasst ist, um durch die äußere Öffnung (den äußeren Muttermund) des Gebärmutterhalses eingeführt zu werden, und ein zweites Ende, das ein Griffelement zum Manipulieren der Röhre beinhaltet, aufweist;
- ein Messelement zum Bestimmen der Position des ersten Endes der Röhre im Gebärmutterhals,
wobei das erste Ende einen Probenentnahmekopf beinhaltet, der angepasst ist, um biologisches Material zu entnehmen, einschließlich extrazelluläre Nukleinsäuren und Schleim, und wobei das Messelement angepasst ist, um den Umfang der Einführung und die transzervikale Position des ersten Endes zu bestimmen, um den Standort der Entnahme der transzervikalen Probe zu optimieren.

11. Verfahren nach einem der Ansprüche 1 bis 10, wobei die Zellen und/oder Zellkerne durch Zentrifugation von den extrazellulären Nukleinsäuren getrennt werden.

12. Verfahren nach einem der Ansprüche 1 bis 11, wobei die Probe in der 5. bis 18. Woche der Schwangerschaft entnommen wurde.

13. Verfahren nach einem der Ansprüche 1 bis 12, das das Entnehmen der Probe von der schwangeren Frau umfasst.

## Revendications

1. Procédé d'enrichissement d'acides nucléique foetaux provenant d'une femelle enceinte, ce procédé comprenant
i) l'obtention d'un échantillon cervical, et
ii) l'enrichissement d'acides nucléiques extracellulaires contenant des acides nucléiques foetaux par séparation de cellules et/ou de noyaux de cellules à partir des acides nucléiques extracellulaires dans l'échantillon.

2. Procédé d'analyse du génotype d'un foetus au niveau d'un locus voulu, ce procédé comprenant
i) l'obtention d'un échantillon cervical,
ii) la séparation de cellules et/ou de noyaux de cellules à partir des acides nucléiques extracellulaires contenant des acides nucléique foetaux dans l'échantillon, et
iii) l'analyse des acides nucléiques extracellulaires pour le génotype d'un foetus au niveau d'un locus voulu.

3. Procédé selon la revendication 3, dans lequel les acides nucléiques sont analysés pour une anomalie génétique liée à un état pathologique ou à une prédisposition à celui-ci.

4. Procédé de détermination du sexe d'un foetus, ce procédé comprenant
i) l'obtention d'un échantillon cervical,
ii) la séparation des cellules et/ou des noyaux de cellules à partir des acides nucléiques extracellulaires contenant des acides nucléique foetaux dans l'échantillon, et
iii) l'analyse des acides nucléiques foetaux pour déterminer le sexe du foetus.

5. Procédé de détermination du père d'un foetus, ce procédé comprenant
i) l'obtention d'un échantillon cervical,
ii) la séparation des cellules et/ou des noyaux de cellules à partir des acides nucléiques extracellulaires contenant des acides nucléique foetaux dans l'échantillon,
iii) la détermination du génotype du candidat à la paternité au niveau d'au moins un locus,
iv) l'analyse des acides nucléique foetaux pour déterminer le génotype du foetus au niveau d'au moins un desdits locus, et
v) la comparaison des génotypes de iii) et iv) pour déterminer la probabilité selon laquelle le candidat à la paternité est le père biologique du foetus.

6. Procédé selon l'une quelconque des revendications 1 à 5, dans lequel au moins une partie des acides nucléiques foetaux dépasse 500 nucléotides en longueur.

7. Procédé selon l'une quelconque des revendications 1 à 6, dans lequel l'échantillon cervical est un échantillon transcervical.

8. Procédé selon l'une quelconque des revendications 1 à 7, dans lequel l'échantillon est obtenu entre l'os interne et l'os externe du col de l'utérus.

9. Procédé selon la revendication 7 ou la revendication 8, dans lequel on obtient l'échantillon plus près de l'os interne du col de l'utérus.

10. Procédé selon l'une quelconque des revendications 1 à 9, dans lequel l'échantillon est obtenu à l'aide d'un dispositif conçu pour l'échantillonnage transcervical de matières biologiques à partir d'une femelle enceinte, ce dispositif comprenant :
- un tube allongé d'insertion comportant une première extrémité conçue pour l'insertion à travers l'orifice externe (os externe) du col de l'utérus et une deuxième extrémité comprenant un moyen formant une poignée permettant de manipuler ledit tube ;
- un moyen de mesure permettant de déterminer la position de ladite première extrémité dudit tube dans le col de l'utérus,
ladite première extrémité comprenant une tête d'échantillonnage conçue pour recueillir de la matière biologique contenant des acides nucléiques extracellulaires et du mucus, et ledit moyen de mesure étant conçu pour déterminer l'ampleur de l'insertion et la position transcervicale de ladite première extrémité afin d'optimiser le site transcervical d'échantillonnage.

11. Procédé selon l'une quelconque des revendications 1 à 10, dans lequel les cellules et/ou les noyaux de cellules sont séparés des acides nucléiques extracellulaires par centrifugation.

12. Procédé selon l'une quelconque des revendications 1 à 11, dans lequel l'échantillon est obtenu dans entre 5 à 18 semaines de grossesse.

13. Procédé selon l'une quelconque des revendications 1 à 12, qui comprend l'obtention de l'échantillon à partir de la femelle enceinte.
